# EUROPEAN PATENT APPLICATION

(11) **EP 2 687 231 A1**
(43) Date of publication of application: **22.01.2014**
(21) Application number: 12760444.5
(22) Date of filing: 16.03.2012
(51) Int. Cl.: A61K 39/395, A61P 1/18, A61P 35/00, A61P 35/04, C12Q 1/02, G01N 33/543, G01N 33/574, G01N 33/577, C07K 14/705, C07K 16/28, C07K 16/46, C12N 15/09

(54) **COMPOSITION FOR TREATMENT AND DIAGNOSIS OF PANCREATIC CANCER**

(30) Priority: 18.03.2011 JP 2011061362
(71) Applicant: Kagoshima University, Kagoshima-shi, Kagoshima 890-8580 (JP)
(72) Inventor: TAKAO, Sonshin, Kagoshima-shi Kagoshima 890-8580 (JP); MATSUYAMA, Takami, Kagoshima-shi Kagoshima 890-8580 (JP)
(74) Representative: Denison, Christopher Marcus
(86) International application number: PCT/JP2012/057667
(87) International publication number: WO 2012/128377

(57) **Abstract**

This invention relates to a pharmaceutical composition for inhibiting the growth and/or metastasis of invasive pancreatic cancer in a subject, comprising a molecular-targeted anticancer agent and a pharmaceutically acceptable carrier, wherein the molecular-targeted anticancer agent is a conjugate of a toxin or cytotoxic agent and an antibody or fragment thereof which immunologically and specifically binds to a cell-surface folate receptor β (FRβ) protein of an FRβ (+) macrophage that exists around pancreatic cancer cells at the invasive front, and to a diagnostic agent and kit for determining the degree of malignancy of pancreatic cancer or the presence of invasive pancreatic cancer, characterized by determining that the cancer tissue is invasive and metastatic when FRβ (+) macrophage is distributed around pancreatic cancer cells at the invasive front.

## Description

### Technical Field

The present invention relates to a pharmaceutical composition for inhibiting the growth and/or metastasis of pancreatic cancer in a subject.

The present invention also relates to application to histological diagnosis and image-diagnosis of invasive pancreatic cancer, specifically to a diagnostic composition.

### Background Art

Pancreatic cancer is difficult to diagnose in its early stages, and it is one of cancers having no ways to effectively diagnose. It is estimated that pancreatic cancer occurs in those aged 40 to 60. It was deduced in a recent report that it takes about a decade for the first cancer cell to give rise to the parental clone, which subsequently becomes a metastatic subclone within 5 years, and then metastasis to other organs occurs within 1 to 2 years thereafter (Non-Patent Document 1).

From a therapeutic aspect, pancreatic cancer responds poorly to currently available anticancer agents, and no effective treatment techniques have been established, although surgery and radiation treatments have been performed.

About 80% of patients with pancreatic cancer are diagnosed with terminal cancer of stage IV, and the 5-year survival rate is as low as about 10% or less. In addition, pancreatic cancer easily infiltrates into large vessels, such as the superior mesenteric artery, aorta, or inferior vena cava, and thus causes metastasis to organs such as liver and lung. In addition to the difficulty of early diagnosis, such cancer infiltration and metastasis make treatment of pancreatic cancer difficult. In the case of patients with inoperable pancreatic cancer, for example, the 50% survival after chemotherapy is reported to be 4.4 months for patients treated with 5-FU and 5.7 months for patients treated with gemcitabine (Non-Patent Document 2).

Meanwhile, the present inventors have reported that a folate receptor β (FRβ) is expressed specifically in infiltrating macrophages at inflammation sites or cancer tissues (Patent Document 1, Patent Document 2, Non-Patent Document 3, and Non-Patent Document 4). Such macrophages are referred to as "tumor-associated macrophages," and have the properties that they release angiogenesis factors and inhibit tumor immunity. Patent Document 1 discloses a therapeutic agent for solid cancer comprising, as an active ingredient, a conjugate of an FRβ-binding antibody and a cytotoxin or cytotoxic agent, and it further describes solid cancers, such as malignant glioma or breast cancer, at which tumor-associated macrophages accumulate (Patent Document 1). It is also described that FRβ is expressed on the surface of cells associated with inflammatory diseases, such as articular rheumatism or acute myelocytic leukemia, and conjugates of a cytotoxin with an anti-FRβ monoclonal antibody as therapeutic agents for such diseases are also disclosed (Patent Document 2, Non-Patent Document 5, and Non-Patent Document 6). It is also known that FRβ expression is not almost observed in peripheral monocytes or indigenous tissue macrophages.

Folate receptors (FRs) include types α, β, γ, and δ. In particular, it is known that the folate receptor type α (FRα) is present on the surface of epithelial tumor membranes of cancers such as ovarian cancer, breast cancer, colorectal cancer, kidney cancer, and lung cancer, and that the sequence identity between FRα and FRβ proteins is approximately 70% (Patent Document 3 and Patent Document 4).

### Prior Art Documents

### Patent Documents

Patent Document 1: JP 2010-77026 A
Patent Document 2: JP 2005-103250 A
Patent Document 3: JP 2009-521206 A
Patent Document 4: JP 2010-503386 A

### Non-Patent Documents

Non-Patent Document 1: Yachida, S. et al., Nature 467: 1115-1117, 2010
Non-Patent Document 2: Burris, H. A. III et al., J. Clin. Oncol. 15: 2403-2413, 2007
Non-Patent Document 3: Puig-Kroger, A. et al., Cancer Res. 69: 9395-9403, 2009
Non-Patent Document 4: Tanaka, M. et al., Clin. Exp. Immunol. 154: 38-47, 2008
Non-Patent Document 5: Nagai, T. et al., Arthritis Rheum. 54: 3126-3134, 2006
Non-Patent Document 6: Nagayoshi, R. et al., Arthritis Rheum. 52: 2666-2675, 2005

### Summary of the Invention

It is an object of the present invention to provide a composition for use in treatment of invasive pancreatic cancer, characterized by inhibiting the growth and/or metastasis of pancreatic cancer, which is difficult to diagnose and treat and thus has the poorest prognosis among other cancers.

It is another object of the present invention to provide a composition or kit for use in histological diagnosis or image diagnosis intended to determine the presence or absence of cancer in pancreatic tissue.

The present invention includes the following.
(1) A pharmaceutical composition for inhibiting the growth and/or metastasis of invasive pancreatic cancer in a subject, comprising a molecular-targeted anticancer agent and a pharmaceutically acceptable carrier, wherein the molecular-targeted anticancer agent is a conjugate of a toxin or cytotoxic agent and an antibody or fragment thereof which immunologically and specifically binds to a cell-surface folate receptor β (FRβ) protein of an FRβ (+) macrophage that exists around pancreatic cancer cells at the invasive front.
(2) The pharmaceutical composition according to (1), wherein the antibody is a monoclonal antibody, a polyclonal antibody, a chimeric antibody, a single chain antibody, a multispecific antibody, or a fragment thereof.
(3) The pharmaceutical composition according to (1) or (2), wherein the antibody is a human antibody or a humanized antibody.
(4) The pharmaceutical composition according to any of (1) to (3), wherein the antibody or a fragment thereof is at least one of (a) to (d) below:
   (a) an antibody or fragment thereof, in which the amino acid sequences of CDRL1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
   (b) an antibody or fragment thereof, in which the amino acid sequences of CDRL1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
   (c) an antibody or fragment thereof, in which the amino acid sequences of CDRL1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ; and
   (d) an antibody or fragment thereof, in which the amino acid sequences of CDRL1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively.
(5) The pharmaceutical composition according to any of (1) to (3), wherein the antibody or fragment thereof binds specifically to an epitope of a region selected from the group consisting of the amino acids 27-65, the amino acids 130-166, and the amino acids 206-233 of the amino acid sequence of SEQ ID NO: 1 of human FRβ protein.
(6) The pharmaceutical composition according to any of (1) to (5), wherein the toxin is a bacterium-derived toxin.
(7) The pharmaceutical composition according to (6), wherein the bacterium-derived toxin is a Pseudomonas toxin, a diphteria toxin, or a staphylococcus toxin.
(8) The pharmaceutical composition according to any of (1) to (7), wherein the molecular-targeted anticancer agent is an immunotoxin.
(9) The pharmaceutical composition according to any of (1) to (5), wherein the cytotoxic agent is an antitumor agent, a tumor growth inhibitor, an apoptosis inducer for tumor cell, or a radioactive nuclide.
(10) The pharmaceutical composition according to any of (1) to (9), wherein the metastasis is lymph node metastasis or hematogenous metastasis.
(11) The pharmaceutical composition according to any of (1) to (10), wherein the subj ect is a human.
(12) A method for examining the degree of malignancy of pancreatic cancer or the presence of invasive pancreatic cancer, comprising: bringing a pancreatic cancer tissue sample of a subject into contact with a labeled or non-labeled antibody or fragment thereof that specifically binds to the cell-surface FRβ protein of the FRβ (+) macrophage; measuring whether the FRβ (+) macrophage exists around the pancreatic cancer tissue at the invasive front based on the formation of a conjugate of the FRβ protein and the antibody or fragment thereof; and determining that the cancer tissue is invasive and metastatic when the FRβ (+) macrophage is distributed around pancreatic cancer cells at the invasive front.
(13) The method according to (12), wherein the antibody is a monoclonal antibody or fragment thereof.
(14) The method according to (12) or (13), wherein the antibody or fragment thereof is at least one of (a) to (e) below:
   (a) an antibody or fragment thereof, in which the amino acid sequences of CDR1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
   (b) an antibody or fragment thereof, in which the amino acid sequences of CDR1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
   (c) an antibody or fragment thereof, in which the amino acid sequences of CDR1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ;
   (d) an antibody or fragment thereof, in which the amino acid sequences of CDR1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively; and
   (e) an antibody or fragment thereof binding specifically to an epitope of a region selected from the group consisting of the amino acids 27-65, the amino acids 130-166, and the amino acids 206-233 of the amino acid sequence of SEQ ID NO: 1 of human FRβ protein.
(15) A diagnostic agent or kit for determining the degree of malignancy of pancreatic cancer or the presence of invasive pancreatic cancer, comprising an antibody or fragment thereof which immunologically and specifically binds to a cell-surface FRβ protein of the FRβ (+) macrophage that exists around pancreatic cancer cells at the invasive front.
(16) A diagnostic agent or kit for image-diagnosing the presence of invasive pancreatic cancer in a subject, comprising a conjugate of a label and an antibody or fragment thereof that immunologically and specifically binds to a cell-surface FRβ protein of a FRβ (+) macrophage.
(17) The diagnostic agent or kit according to (10), wherein the label is a fluorophore, pigment, or radioactive isotope.
(18) The diagnostic agent or kit according to any of (15) to (17), wherein the antibody or fragment thereof is at least one of (a) to (e) below:
   (a) an antibody or fragment thereof, in which the amino acid sequences of CDR1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
   (b) an antibody or fragment thereof, in which the amino acid sequences of CDR1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
   (c) an antibody or fragment thereof, in which the amino acid sequences of CDR1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ;
   (d) an antibody or fragment thereof, in which the amino acid sequences of CDR1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively; and
   (e) an antibody or fragment thereof binding specifically to an epitope of a region selected from the group consisting of the amino acids 27-65, the amino acids 130-166, and the amino acids 206-233 of the amino acid sequence of SEQ ID NO: 1 of human FRβ protein.

This description includes all or part of the contents as disclosed in the description and/or drawings of Japanese Patent Application No. 2011-061362, from which the present application claims priorities.

### Brief Description of the Drawings

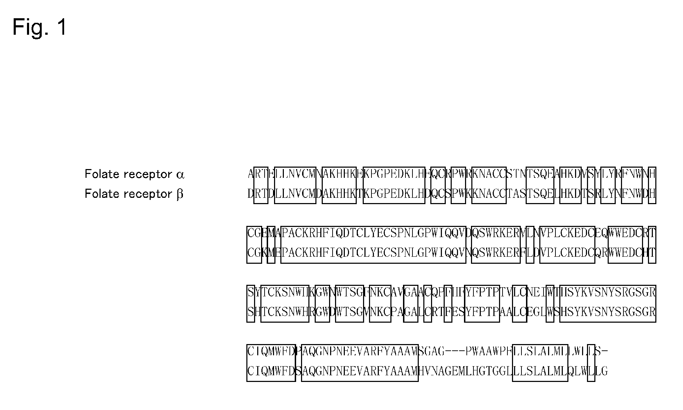

Fig. 1 shows the alignment of the amino acid sequences of human folate receptor α (FRα) and human FRβ.

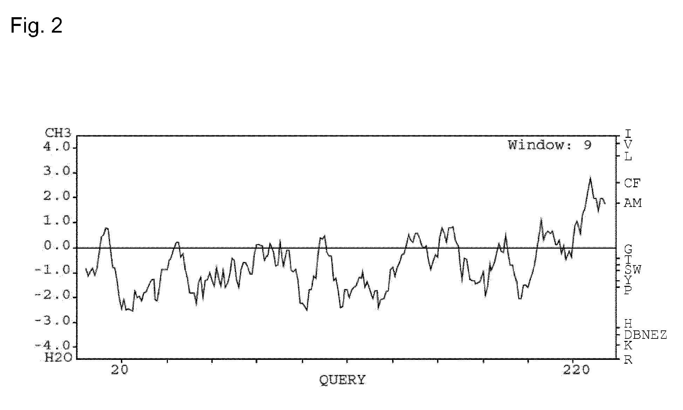

Fig. 2 shows the graph representing the Kyte-Doolittle hydrophilicity-hydrophobicity prediction for human FRβ protein.

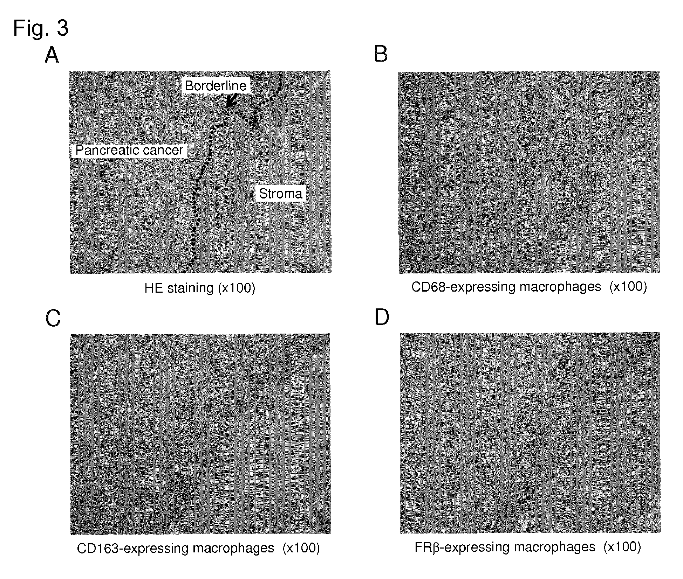

Fig. 3 shows the distribution of the CD68-expressing macrophage (B), the CD163-expressing macrophage (C), and the FRβ-expressing macrophage (D) in human pancreatic cancer tissue (A). Fig. 3A shows hematoxylin-eosin (HE) staining, and Figs. 2B, 2C, and 2D show the staining with anti-CD68 antibody, anti-CD163 antibody, and anti-FRβ antibody, respectively.

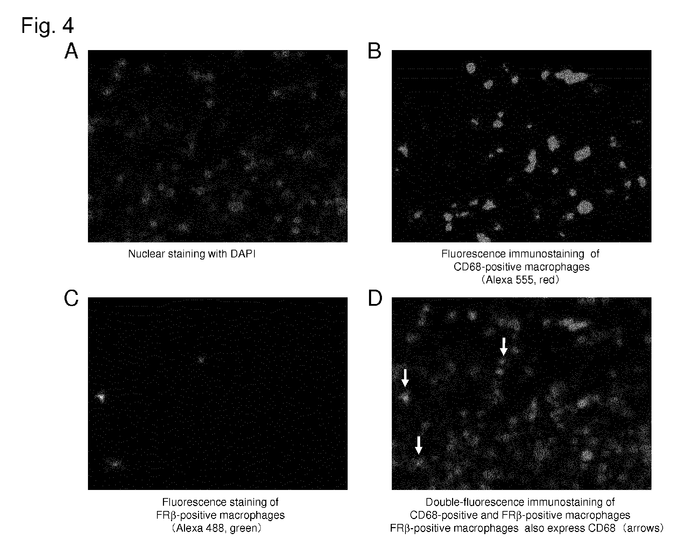

Fig. 4 shows the staining of macrophages at the invasive front of pancreatic cancer: (A): nuclear staining with DAPII; (B): fluorescence staining of CD68-positive macrophage with Alexa555 (red); (C): fluorescence staining of FRβ-positive macrophage with Alexa488 (green); and (D): double-fluorescence staining of CD68-positive macrophage and FRβ-positive macrophage.

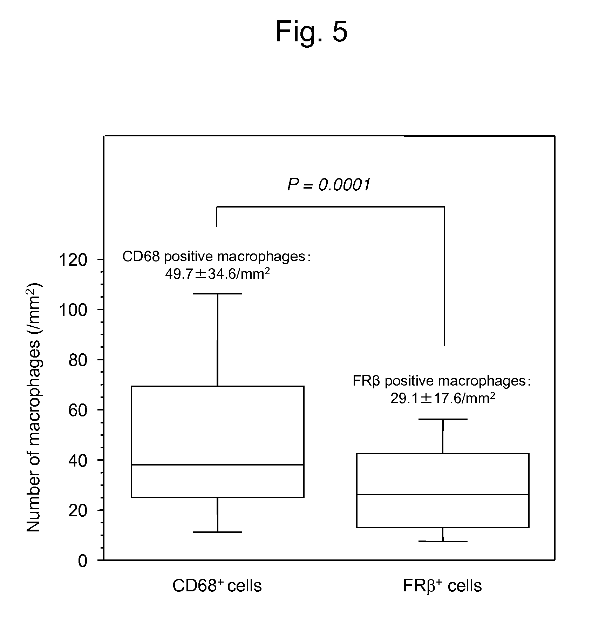

Fig. 5 shows a comparison of the number of CD68-positive macrophages invading pancreatic cancer with that of FRβ-positive macrophages.

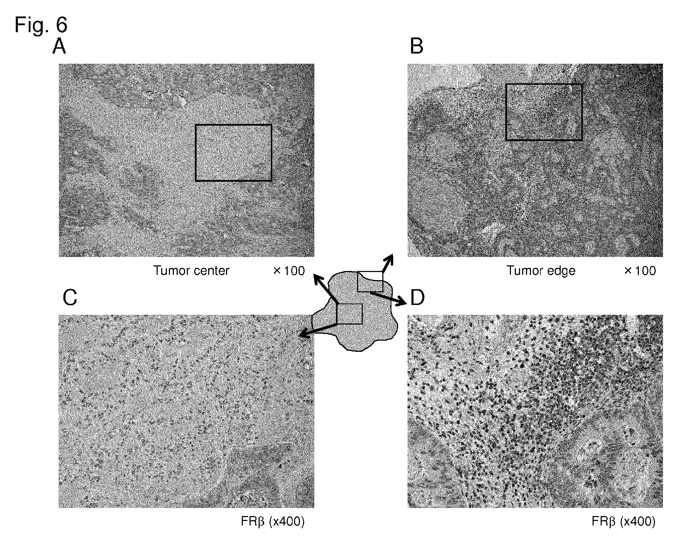

Fig. 6 shows the detection of the FRβ-expressing macrophage of human pancreatic cancer transplanted into nude mice, wherein the macrophage was stained with anti-mouse FRβ antibody. Fig. 6A and Fig. 6C show the FRβ expression in FRβ-expressing macrophages at the center of the transplanted tumor, and Fig. 6B and Fig. 6D show the FRβ expression in FRβ-expressing macrophages at the edge of the transplanted tumor.

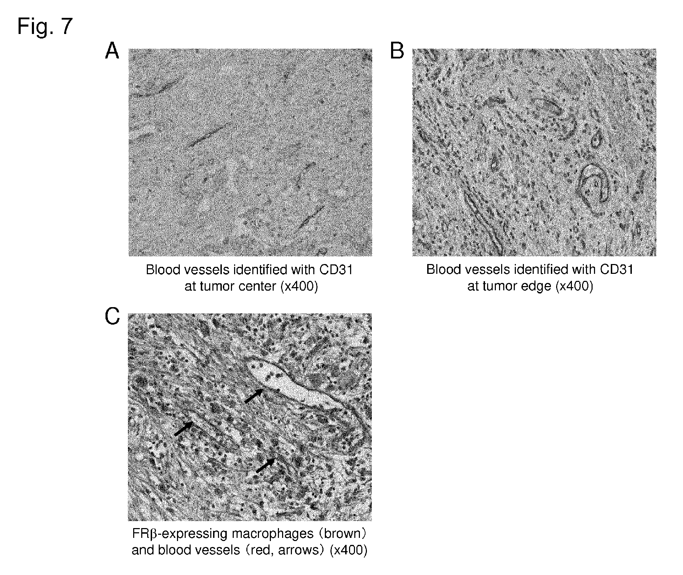

Fig. 7 shows FRβ-expressing macrophages distributed around newborn blood vessels in tumor at the front of human pancreatic cancer (A, B, and C). Fig. 7A shows the results of evaluation of the vessels with CD31 at the tumor center; Fig. 7B shows the results of evaluation of the vessels with CD31 at the tumor edge; and Fig. 7C shows FRβ-expressing macrophages (brown) and the vessels (red, indicated by arrows). The magnification is x400.

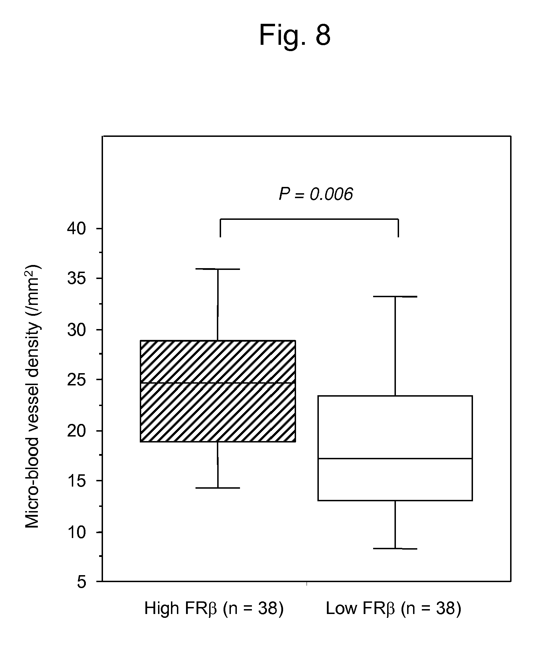

Fig. 8 shows the correlation between high FRβ expression in FRβ (+) macrophage and the high density of newborn blood vessels in tumor.

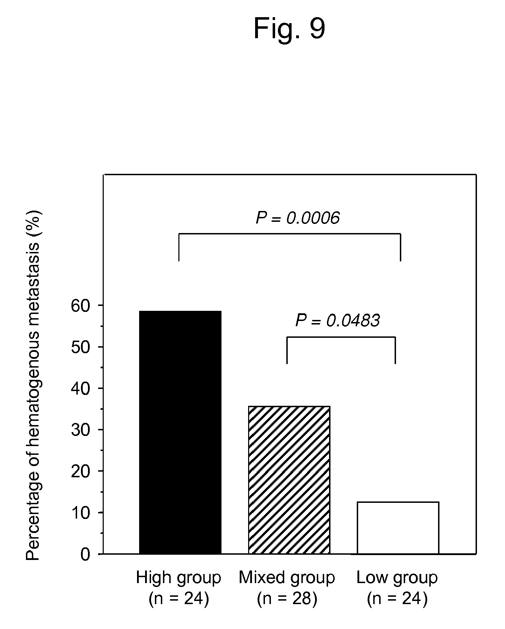

Fig. 9 shows the correlation between high FRβ expression in FRβ (+) macrophages and hematogenous metastasis.

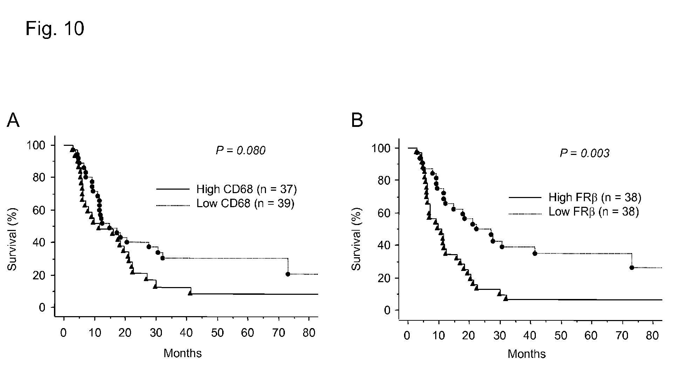

Fig. 10 shows the correlation between the duration of post-operative survival of a human subject with pancreatic cancer and the FRβ-expression in FRβ-positive macrophage. Fig. 10A shows a comparison of survival rates between a high expression group of CD68-positive macrophage and a low expression group of CD68-positive macrophage; and Fig. 10B shows a comparison of survival rates between a high expression group of FRβ-positive macrophage and a low expression group of FRβ-positive macrophage.

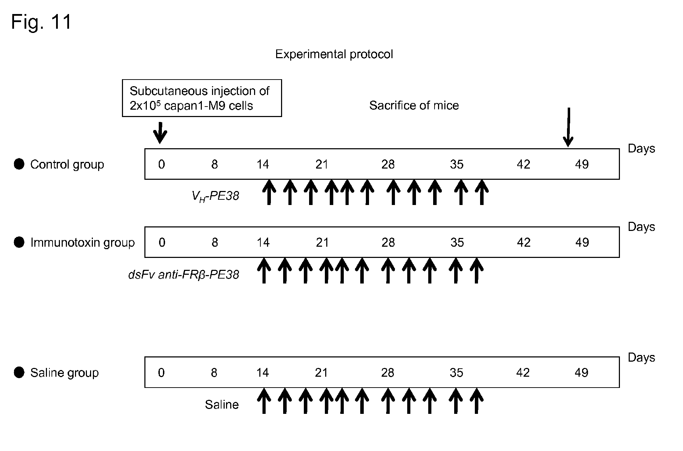

Fig. 11 shows the experimental design for examining the inhibition of the growth of recombinant immunotoxin (dsFv anti-FRβ-PE38) in nude mice into which human pancreatic cancer (the capan1-M9 strain) had been transplanted into the skin. The VH-PE38 group and the saline group were provided as control groups. Mice were sacrificed on days indicated by arrows.

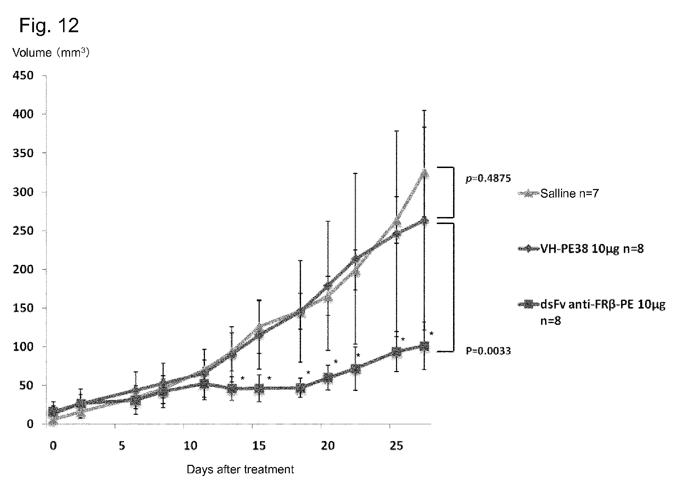

Fig. 12 shows a graph indicating changes in tumor volume in mice treated in accordance with the experimental design recited in Fig. 11 with the elapse of time.

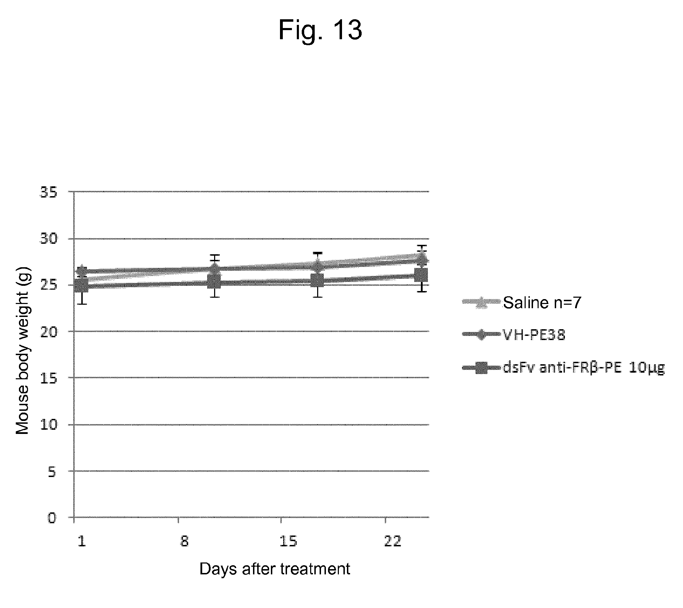

Fig. 13 shows a graph indicating that mouse body weights do not substantially vary among 3 groups when treatment is performed in accordance with the experimental design recited in Fig. 11.

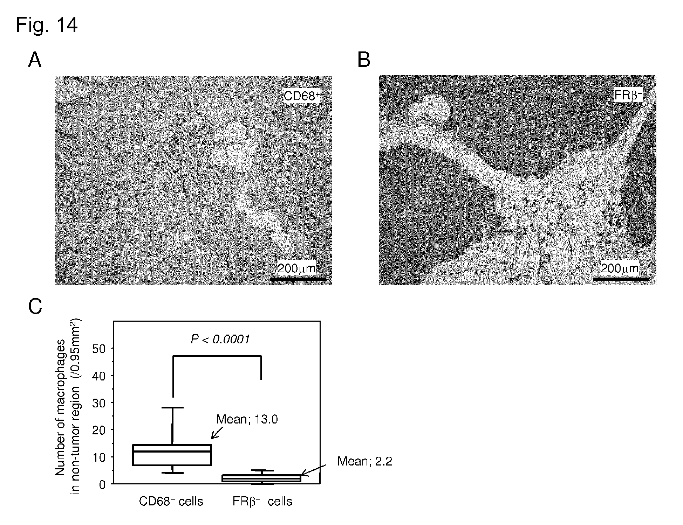

Fig. 14 shows the results of detection of CD68-expressing macrophage and FRβ-expressing macrophage in normal human pancreatic tissue. "A" and "C" indicate that the CD68-positive macrophage expression was observed, and "B" and "C" indicate that the FRβ-positive macrophage expression was not observed with a significant difference.

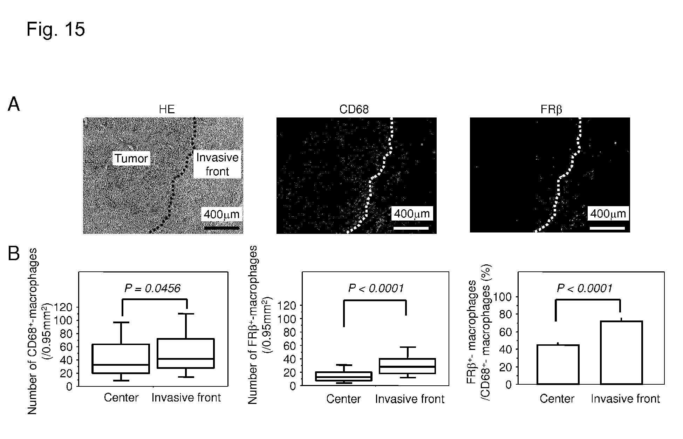

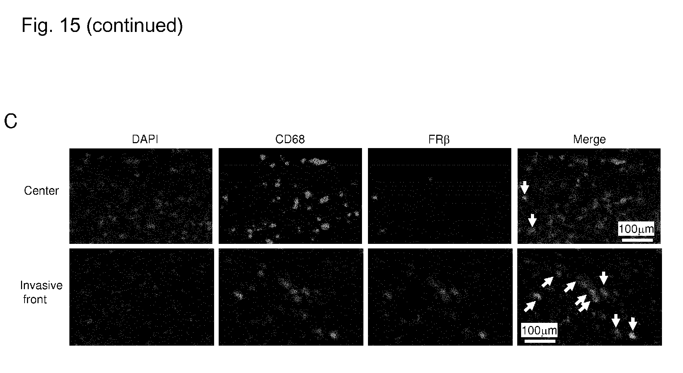

Fig. 15 shows the distribution of CD68 (+) macrophage and FRβ (+) macrophage in neoplastic human pancreatic tissue detected via fluorescence immunostaining. "A", "B", and "C" show the results of comparison of the distribution of CD68 (+) macrophages and FRβ (+) macrophages between the invasive front and the center of pancreatic cancer. In Fig. 15, the color red indicates CD68 (+) macrophages and the color green indicates FRβ (+) macrophages.

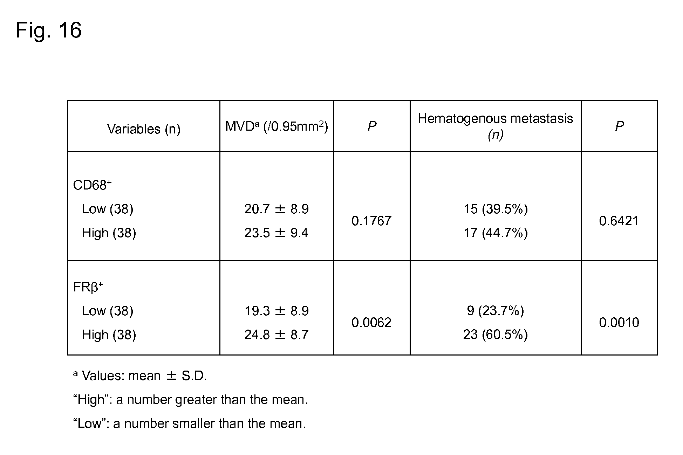

Fig. 16 shows the comparison of CD68-high expression and -low expression and FRβ-high expression and -low expression in CD68 (+) macrophages and in FRβ (+) macrophages in human pancreatic cancer tissue, with the number of micro-blood vessels in tumor and hematogenous metastasis in patients with pancreatic cancer.

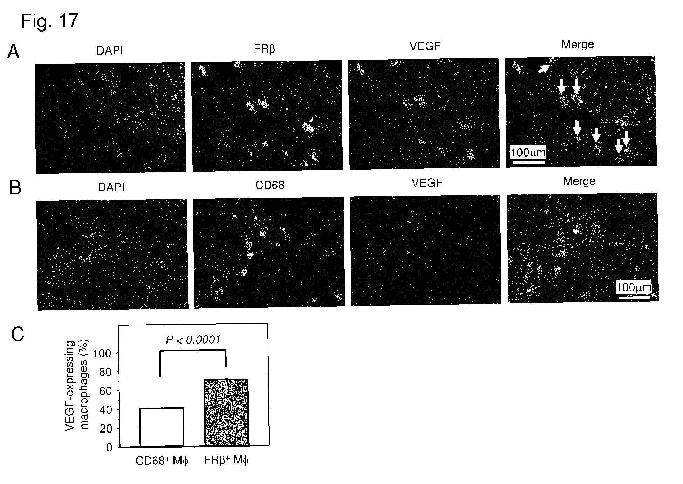

Fig. 17 shows the results of expression analysis of angiogenesis factor (VEGF) in CD68 (+) macrophages and in FRβ (+) macrophages in human pancreatic cancer tissue. "A" indicates the expression of VEGF in the FRβ (+) macrophages, "B" indicates the expression of VEGF in the CD68 (+) macrophages, and "C" shows a graph representing quantified results of A and B.

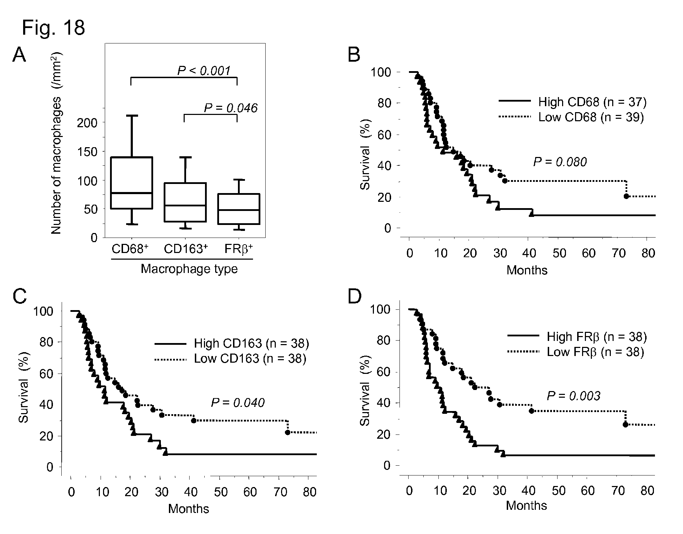

Fig. 18 shows the comparison of the numbers of different types of invasive macrophages (i.e., CD68, CD163, and FRβ) (A) and survival curves for patients with pancreatic cancer depending on macrophage types (B, C, and D).

### Mode for Carrying out the Invention

The present invention will be described in more detail.

### <Definition>

The term "folate receptors β" or "FRβ" used herein refers to a receptor protein expressed on the surface of tumor-associated macrophages in a subject. In the present invention, such macrophages are M1 and/or M2 macrophages distributed and localized around invasive pancreatic cancer.

The term "subject" used herein refers to, for example, mammalian animals, such as primates including humans, livestock animals such as cattle, pigs, horses, goats, and sheep, and pet animals such as dogs and cats. A preferable subject is a human.

As used herein, the term "an antibody binding immunologically and specifically to FRβ protein" refers to an antibody which does not bind to proteins other than the FRβ protein or naturally-occurring variants thereof or does not substantially bind to other proteins.

The term "metastasis" used herein means that pancreatic cancer becomes invasive and causes lymphatic invasion or vascular invasion, and the pancreatic cancer is colonized in organs other than lymph nodes or pancreas to develop tumors.

### <FRβ antibody>

The term "an antibody binding immunologically and specifically to the cell-surface FRβ protein of the folate receptor β (FRβ)(+) macrophage located around pancreatic cancer cells at the invasive front" as used herein refers to an antibody, or a fragment thereof, that is capable of recognizing and binding to the FRβ protein or a fragment thereof, regardless of a type or form of an antibody, as described below. Such antibody enables specific binding to the cell-surface FRβ protein of the FRβ (+) macrophage located around the pancreatic cancer cells at the invasive front.

In the present invention, the above antibody binds to the FRβ macrophage via immunological reaction; however, such antibody does not substantially bind to proteins other than FRβ or variant proteins thereof having 90% or higher, preferably 95% or higher, and more preferably 98% or higher sequence identity with FRβ.Accordingly, such antibody does not bind to FRα, which is one of FR isoforms (e.g., the amino acid sequence identity between human FRα and human FRβ is about 70% (JP 2008-500025 A)).

The antibody according to the present invention may be of any immunoglobulin (Ig) class (e.g., IgA, IgG, IgE, IgD, IgM, or IgY) and any subclass (e.g., IgG1, IgG2, IgG3, IgG4, IgA1, or IgA2). Also, an immunoglobulin light chain may be the κ or λ chain.

Specifically, the antibody according to the present invention is, for example, an antibody having a complete structure of the aforementioned class or subclass, or a fragment of the antibody, such as a recombinant antibody, a single chain antibody (scFv), a multispecific antibody (e.g., a double-specific antibody, a Diabody, Triabody, ScDb (single chain diabody), and dsFv-dsFv), a chimeric antibody, a humanized antibody, or a human antibody, or a fragment of any thereof.

An antibody fragment that can be used in the present invention is capable of binding to an epitope of the FRβ protein antigen comprising 7 or more, and preferably at least 8 to 12 continuous amino acids.

Examples of antibody fragments include Fab, Fab', F(ab')₂, Fv, Fd, and Fabc. Antibody fragments can be prepared by methods known in the art. For example, antibody fragments can be prepared by digesting antibody molecules with a protease such as papain or pepsin or by known genetic engineering techniques.

Hereafter, methods for preparing antibodies used in the present invention are described in detail.

In order to prepare antibodies that can be used in the present invention, at the outset, the FRβ protein to be used as an immunogen (i.e., an antigen) or a fragment of the FRβ protein is prepared.

A fragment comprises a sequence comprising 7-10 or more, such as 11-25, continuous amino acids. The origin of the FRβ protein that can be used as an immunogen is not particularly limited, provided that it is capable of inducing an antibody that can bind specifically to a target FRβ.

In order to induce an antibody that can bind specifically to FRβ, FRα (which has an approximately 70% sequence identity with FRβ at the protein level) is aligned with FRβ, and a (poly)peptide comprising a partial sequence of FRβ protein having a low identity between the sequence portions comprising approximately 7-20 continuous amino acids can be selected as the immunogen. In such a case, it is preferable that an FRβ protein surface structure is predicted with the utilization of, for example, the results of hydrophilicity/hydrophobicity prediction using the Kyte-Doolittle method or secondary structure prediction based on amino acid sequences by the Chou-Fasman method (Biochemistry 1974, 13: 222-244) to select the (poly)peptide sequence exposed on the protein surface as the immunogen.

Fig. 1 and Fig. 2 show the FRβ-FRα alignment and the results of the Kyte-Doolittle hydrophilicity/hydrophobicity prediction of FRβ, respectively. FRβ is a GPI-anchored protein (M. N. Prioleau et al., EMBO. J. 1999, 18: 4035-4048). The signal sequence of FRβ comprises a hydrophobic region of about 30 C-terminal amino acid residues. Specifically, this region comprises a C-terminal sequence HVNAG ··QLWLLG as shown in Fig. 1. As shown in Fig. 2, the C-terminal sequence is a highly hydrophobic region. In general, the GPI-anchored protein binds to a membrane via a GPI-anchor, and it does not have a transmembrane domain and an intracellular domain. According to Fig. 2, however, FRβ is a highly hydrophilic protein as a whole, and a protein region is exposed to the outside of the cell. On the basis of the amino acid sequence alignment of FRβ (SEQ ID NO: 1) and FRα (SEQ ID NO: 3) shown in Fig. 1, fragments exhibiting low sequence identity with each other, for example, a peptide sequence comprising at least 8 to 12 continuous amino acid residues (e.g., about 10 to 20 continuous amino acid residues) in a region selected from the group consisting of a region comprising amino acids 27 to 65, a region comprising amino acids 130 to 166, and a region comprising amino acids 206 to 233 in the amino acid sequence as shown in SEQ ID NO: 1 can be selected, and an antibody binding specifically to such peptide; i.e., an antibody that binds to such peptide but does not substantially bind to other protein such as FRα, can be prepared.

The information concerning the amino acid or nucleotide sequence of FRβ of mammalian animals, including humans, of the FRβ protein used for preparing the antibody of the present invention or a fragment of FRβ is based on the amino acid or nucleotide sequence (SEQ ID NO: 1 or SEQ ID NO: 2) of, for example, human FRβ, and such information is available from the databanks of proteins, genes, and the like known in the art, such as GenBank (NCBI, U.S.A.) or EMBL (EBI, Europe). The accession numbers of the amino acid sequence and the nucleotide sequence of FRβ registered at the GenBank include some known variants, and examples thereof include NM_000803 (transcript variant 1), NM_001113534 (transcript variant 2), and NM_001113535 (transcript variant 3). The FRβ protein or a fragment thereof can be produced via peptide synthesis or genetic recombination techniques known in the art based on the sequence information above.

FRβ variants or orthologs of the same or different animal species can be searched with the use of the algorithm described by, for example, Karlin and Altschul (1993, Proc. Natl. Acad. Sci., U.S.A., 90: 5873-5877) or the modified algorithm (Karlin and Altschul, 1990, Proc. Natl. Acad. Sci., U.S.A., 87: 2264). Such algorithm is incorporated into the NBLAST and XBLAST programs described in Altschul et al. (1990, J. Mol. Biol., 215: 403). In addition, Gapped BLAST described by Altschul et al. (1997, Nucleic Acids Res. 25: 3389) can be employed in order to obtain a gap-introduced alignment.

Peptide synthesis can be carried out by a liquid-phase method or a solid-phase method. While a difference between such techniques lies only in the use of a solid-phase, the solid-phase method is more advantageous in terms of easy collection of products. Thus, the solid-phase method is effectively employed. Both methods each comprise synthesizing large number of peptides comprising approximately 5 to 10 (protective) amino acids constituting a protein, extending the peptides in a stepwise manner to synthesize polypeptides, and removing the protective groups in the end to produce a target protein, followed by purification. The method of peptide synthesis is described in, for example, Seikagaku Jikken Kouza 1 (Lecture Course for Biochemical Experiment) vol. 1, Tanpakushitsu no Kagaku (Protein Chemistry) IV, Kagaku shushoku to Peptide Gousei (Chemical Modification and Peptide Synthesis), the Japanese Biochemical Society (ed.), Tokyo Kagaku Dojin Co. Ltd.

According to genetic engineering techniques, for example, DNA encoding the FRβ protein is ligated to an adequate vector, the resulting vector is introduced into an adequate host cell for transformation, and the host cell is cultured in an adequate medium to produce the FRβ protein. These techniques are well-known to a person skilled in the art, and vectors, host cells, and transformation, culture, protein purification, and other techniques are described in, for example, Sambrook et al., Molecular Cloning: A Laboratory Manual, vol. 2, Cold Spring Harbor Laboratory Press, 1989; and Ausubel et al., Current Protocols in Molecular Biology, John Wiley & Sons, 1998.

Examples of antibody-producing cells include: insect cells such as Spodoptera frugiperda cells; yeast cells such as *Saccharomyces cerevisiae* and *Schizosaccharomyces pombe;* and mammalian cells such as Chinese hamster ovary (CHO) cells, hamster embryonic kidney cells, human embryonic kidney 293 cells, normal dog kidney cells, normal cat kidney cells, monkey kidney cells, African green monkey kidney cells, COS cells, non-tumor mouse muscle myoblasts (G8), fibroblasts, myeloma cells, mouse NIH/3T3 cells, LMTK cells, mouse sertoli cells, human cervical cancer cells, buffalo rat liver cells, human lung cells, human liver cells, mouse breast cancer cells, TRI cells, MRC5 cells, and FS4 cells.

Non-human animals are immunized with the thus-produced FRβ protein or a fragment thereof as the immunogen, and the antibody according to the present invention can be produced by the method described below. Alternatively, DNA encoding the heavy chain and the light chain variable regions encoding such antibody is prepared from mRNA of the spleen cells, lymph cells, or other cells of the immunized non-human animals, and the resulting DNA can be used to synthesize DNA encoding synthetic antibodies of various forms, such as chimeric antibodies.

Examples of antibodies that can be used in the present invention include, but are not limited to, polyclonal antibodies, monoclonal antibodies, recombinant antibodies (e.g., chimeric antibodies, single chain antibodies, multispecific antibodies, and humanized antibodies), and human antibodies.

Polyclonal antibodies can be produced by immunizing mammalian animals, such as rabbits, rats, or mice, with the immunogen prepared in the manner described above and obtaining the antiserum. Specifically, the immunogen is intravenously, subcutaneously, or intraperitoneally administered to mammalian animals together with an adjuvant to enhance the immunogenicity, according to need. Examples of adjuvant that can be used include commercially available Freund's complete adjuvants, incomplete Freund's adjuvants, aluminum salts (Alum) such as ammonium hydroxide, and muramyl peptide (i.e., a type of peptide associated with a bacterial cell wall). Thereafter, immunization is performed 1 to 7 times at intervals of several days to several weeks, the antibody titer is assayed 1 to 7 days after the final immunization via enzyme immunoassay techniques, such as ELISA, and the blood is sampled when the maximal antibody titer is measured, so as to obtain the antiserum. The thus-obtained antiserum may be used as such. Alternatively, the antiserum may be purified before use by applying the antiserum to an affinity column on which the FRβ protein or a peptide fragment thereof has been immobilized (e.g., an agarose gel column) and then recovering antibodies bound to the column.

Monoclonal antibodies can be prepared in the manner described below. Specifically, hybridomas are prepared from the antibody-producing cells obtained from non-human mammalian animals that were immunized in the manner described above (e.g., spleen cells or lymphoid cells) and immortalized myeloma cells by the fusion technique, the hybridomas are subjected to cloning, and clones producing monoclonal antibodies showing specific affinity to the FRβ protein used for immunization or peptide antigen fragments thereof are selected using a medium containing hypoxanthine, aminopterin, and thymidine (i.e., HAT medium). Thus, monoclonal antibodies can be prepared. Hybridomas can be prepared in accordance with, for example, the method of Kohler and Milstein et al. (Nature, 1975, 256: 495-96). Examples of non-human mammalian animals include rodents such as mice and rats. As myeloma cells, cells originating from the animals of the same species with the immunized animals are preferably used, and examples thereof include mouse myeloma cells and rat myeloma cells. Specific examples of mouse myeloma cell lines include P3-NS1/1-Ag4-1, P3-x63-Ag8.653, and Sp2/O-Ag14 cell lines. Antibody-producing cells can be fused to myeloma cells with the use of polyethylene glycol (PEG) having the average molecular weight of about 1,500 or via electroporation.

Chimeric antibodies, recombinant antibodies, single chain antibodies, humanized antibodies, and the like can be produced from DNAs encoding the antibodies derived from hybridomas producing specific monoclonal antibodies prepared from the myeloma cells and the spleen cells from non-human animals that were immunized with the FRβ protein or fragments thereof.

Specifically, total RNA is extracted from the hybridoma cells, mRNAs showing binding affinity for the oligo dT column are recovered from total RNA, cDNA is synthesized, and DNA encoding a particular monoclonal antibody is cloned. Alternatively, DNA encoding an antibody of interest is synthesized via PCR amplification based on the known immunoglobulin gene sequence, and the DNA sequence is determined. The sequences and the positions of the variable regions, complementarity-determining regions (CDRs), and framework regions (FRs) of the antibody heavy (H) chain and light (L) chain of animals, such as humans or mice, can be determined in accordance with, for example, the Kabat's EU numbering index (Kabat E. A. et al., Sequences of Proteins of Immunological Interest, 5th ed., Vol. 1, Bethesda (MD): NIH, 1991).

Methods for preparing recombinant antibodies using genetic engineering techniques are described in greater detail.

Genes encoding monoclonal antibodies are cloned from the prepared hybridomas and integrated into adequate vectors, the resultants are introduced into host cells, such as mammalian cells such as Chinese hamster ovary (CHO) cells, E. coli cells, yeast cells, insect cells, or plant cells, and recombinant antibodies can be produced in the host cells (P. J. Delves., ANTIBODY PRODUCTION ESSENTIAL TECHNIQUES., 1997, WILEY, P. Shepherd and C. Dean., Monoclonal Antibodies., 2000, OXFORD UNIVERSITY PRESS, J. W. Goding., Monoclonal Antibodies: Principles and Practice, 1993 ACADEMIC PRESS). Alternatively, transgenic mice, cattle, goats, sheep, or pigs in which the endogenous gene locus has been substituted with the gene locus of a target antibody are prepared by techniques for producing transgenic animals, the resulting transgenic animals are immunized with the FRβ protein or a peptide fragment thereof as the immunogen, and the antibodies derived from the antibody gene can be obtained from, for example, the blood or milk of such transgenic animals. Some of the above-mentioned transgenic animals are human antibody producing animals such as mice or cattle that lack endogenous antibody genes and possess the human antibody genes. When utilizing such animals, accordingly, complete human antibodies binding to human FRβ can be obtained (e.g., WO 96/9634096, WO 96/33735, and WO 98/24893). When hybridomas prepared from the antibody-producing cells of the animals (e.g., B cells) and myeloma cells are cultured in vitro, further, monoclonal antibodies can be produced in the manner described above.

The monoclonal antibodies prepared can be purified by methods known in the art, such as chromatography involving the use of a protein A or G column, ion exchange chromatography, hydrophobic chromatography, salting out with ammonium sulfate, gel filtration, or affinity chromatography, and such techniques can be performed in adequate combination.

Chimeric antibodies comprise H-chain and L-chain variable regions and constant regions originating from different animal species. For example, chimeric antibodies comprise H-chain and L-chain variable regions originating from mouse antibodies and H-chain and L-chain constant regions originating from human antibodies. DNA encoding such antibodies comprises a nucleotide sequence resulting from substitution of a DNA sequence encoding a constant region in the DNA sequence encoding mouse antibodies with a DNA sequence encoding a human constant region. Chimeric antibodies can be produced by the technique described in, for example, Morrison et al., 1984, Proc. Natl. Acad. Sci., 81: 6851-6855; Neuberger et al., 1984, Nature, 312: 604-608; Takeda et al., 1985, Nature, 314: 452-454.

Humanized antibodies comprise H-chain and L-chain CDRs originating from non-human animals and constant regions and framework regions originating from humans. For example, humanized antibodies comprise H-chain and L-chain CDRs (CDR1, CDR2, and CDR3) originating from mouse antibodies and constant (C) regions and framework regions (FR1, FR2, FR3, and FR4) originating from human antibodies. DNA encoding such antibodies comprises a nucleotide sequence resulting from substitution of DNA sequences encoding the constant regions and the framework regions of the DNA sequence encoding mouse antibodies with DNA sequences encoding the constant regions and the framework regions originating from human antibodies. A technique for preparing humanized antibodies is a so-called CDR grafting technique. Among the antibodies used in the present invention, in particular, antibodies that can be administered to humans can be prepared by the CDR grafting technique (Jones et al., Nature, 321: 522-525, 1986; Reichmann et al., Nature, 332: 323-329, 1988; Presta, Curr. Op. Struct. Biol., 2: 593-596, 1992; Verhoeyen et al., Science, 239: 1534-1536, 1988). Alternatively, complete human antibodies can be produced with the use of non-human animals that produce human antibodies (e.g., mice) or by the phage display technique, as described below.

According to the phage display technique, phages such as filamentous bacteriophages M13 or bacteriophages T7 are used to express fusion proteins of phage coat proteins with foreign polypeptides (e.g., recombinant FRβ antibodies) and present the fusion proteins on the phage surface (e.g., C. Barbas et al., Phage Display: Laboratory Manual, Cold Spring Harbor Laboratory Press, 2001). As phage coat proteins, g3p or g8p in the M13 phage or g10p in the T7 phage can be used to present foreign polypeptides. Examples of recombinant FRβ antibodies include single chain antibodies (scFv) comprising the H chain variable region (VH) bound to the L-chain variable region (VL) through a linker (e.g., (GGGGS)₃), synthetic antibodies such as multispecific antibodies (i.e., antibodies comprising two or more different VHs in combination with two or more different VLs), and (recombinant) H-chain (VH and CH) or L-chain (VL and CL) of human antibodies, or a combination thereof.

Specific examples of the antibodies that can be used in the present invention and produced by the method described above include, but are not limited to, the antibodies described below. In addition to the antibodies described below, recombinant antibodies such as chimeric antibodies, single chain antibodies, or multispecific antibodies comprising the complementarity determining regions of the heavy chain variable region and the light chain variable region of the antibodies can be contained as active ingredients into the composition, kit, and diagnostic agent of the present invention:
an antibody, or a fragment thereof, that binds specifically to a peptide comprising a sequence of at least 8 to 12 continuous amino acid residues, such as about 10 to 20 continuous amino acid residues, of a region selected from the group consisting of a region of amino acids 27 to 65, a region of amino acids 130 to 166, and a region of amino acids 206 to 233 of the amino acid sequence as shown in SEQ ID NO: 1;
an antibody or a fragment thereof, comprising the light chain variable region (VL) of the amino acid sequence as shown in SEQ ID NO: 4 (the corresponding nucleotide sequence: SEQ ID NO: 5) and the heavy chain variable region (VH) of the amino acid sequence as shown in SEQ ID NO: 6 (the corresponding nucleotide sequence: SEQ ID NO: 7), derived from the anti-human FRβ mouse monoclonal antibody 36b;
an antibody or a fragment thereof, comprising the light chain variable region (VL) comprising CDR1, CDRL2, and CDRL3 of the amino acid sequences as shown in SEQ ID NOs: 8, 9, and 10, respectively, and the heavy chain variable region (VH) comprising CDRH1, CDRH2, and CDRH3 of the amino acid sequences as shown in SEQ ID NOs: 11, 12, and 13, respectively, derived from the anti-human FRβ mouse monoclonal antibody 36b;
an antibody or a fragment thereof, comprising the light chain variable region (VL) of the amino acid sequence as shown in SEQ ID NO: 14 (the corresponding nucleotide sequence: SEQ ID NO: 15) and the heavy chain variable region (VH) of the amino acid sequence as shown in SEQ ID NO: 16 (the corresponding nucleotide sequence: SEQ ID NO: 17), derived from the anti-human FRβ mouse monoclonal antibody 94b;
an antibody or a fragment thereof, comprising the light chain variable region (VL) comprising CDR1, CDRL2, and CDRL3 of the amino acid sequences as shown in SEQ ID NOs: 18, 19, and 20, respectively, and the heavy chain variable region (VH) comprising CDR1, CDRH2, and CDRH3 of the amino acid sequences as shown in SEQ ID NOs: 21, 22, and 23, respectively, derived from the anti-human FRβ mouse monoclonal antibody 94b;
an antibody or a fragment thereof, comprising the light chain variable region (VL) of the amino acid sequence as shown in SEQ ID NO: 24 (the corresponding nucleotide sequence: SEQ ID NO: 25) and the heavy chain variable region (VH) of the amino acid sequence as shown in SEQ ID NO: 26 (the corresponding nucleotide sequence: SEQ ID NO: 27), derived from the anti-mouse FRβ rat monoclonal antibody CL5;
an antibody or a fragment thereof, comprising the light chain variable region (VL) comprising CDR1, CDRL2, and CDRL3 of the amino acid sequences as shown in SEQ ID NOs: 28, 29, and 30, respectively, and the heavy chain variable region (VH) comprising CDR1, CDRH2, and CDRH3 of the amino acid sequences as shown in SEQ ID NOs: 31, 32, and 33, respectively, derived from the anti-mouse FRβ rat monoclonal antibody CL5;
an antibody or a fragment thereof, comprising the light chain variable region (VL) of the amino acid sequence as shown in SEQ ID NO: 34 (the corresponding nucleotide sequence: SEQ ID NO: 35) and the heavy chain variable region (VH) of the amino acid sequence as shown in SEQ ID NO: 36 (the corresponding nucleotide sequence: SEQ ID NO: 37), derived from the anti-mouse FRβ rat monoclonal antibody CL10;
an antibody or a fragment thereof, comprising the light chain variable region (VL) comprising CDR1, CDRL2, and CDRL3 of the amino acid sequences as shown in SEQ ID NOs: 38, 39, and 40, respectively, and the heavy chain variable region (VH) comprising CDR1, CDRH2, and CDRH3 of the amino acid sequences as shown in SEQ ID NOs: 41, 42, and 43, respectively, derived from the anti-mouse FRβ rat monoclonal antibody CL10.

The above antibodies can comprise mutation, such as substitution, deletion, or addition (or insertion) of 1 to 3, and preferably 1 or 2 amino acid residues, in the heavy chain variable region, the light chain variable region, the framework region, or the constant region. Specific examples thereof are provided below:
an antibody or a fragment thereof, comprising a sequence having a substitution of glycine 63 of SEQ ID NO: 16 (the nucleotide sequence: ggc) with cysteine (Cys; the nucleotide sequence: tgt) in the amino acid sequence of the light chain variable region (VL) (SEQ ID NO: 14; the corresponding nucleotide sequence: SEQ ID NO: 15) and the amino acid sequence of the heavy chain variable region (VH) (SEQ ID NO: 16; the corresponding nucleotide sequence: SEQ ID NO: 17), derived from the anti-human FRβ mouse monoclonal antibody 94b;
an antibody or a fragment thereof, comprising a sequence having a substitution of glycine 125 of SEQ ID NO: 14 (the nucleotide sequence: gga) with cysteine (Cys; the nucleotide sequence: tgt) in the amino acid sequence of the light chain variable region (VL) (SEQ ID NO: 14; the corresponding nucleotide sequence: SEQ ID NO: 15) and the amino acid sequence of the heavy chain variable region (VH) (SEQ ID NO: 16; the corresponding nucleotide sequence: SEQ ID NO: 17), derived from the anti-human FRβ mouse monoclonal antibody 94b;
an antibody or a fragment thereof, comprising a sequence having a substitution of glycine 63 of SEQ ID NO: 36 (the nucleotide sequence: ggc) with cysteine (Cys; the nucleotide sequence: tgt) in the amino acid sequence of the light chain variable region (VL) (SEQ ID NO: 34; the corresponding nucleotide sequence: SEQ ID NO: 35) and the amino acid sequence of the heavy chain variable region (VH) (SEQ ID NO: 36; the corresponding nucleotide sequence: SEQ ID NO: 37), derived from the anti-mouse FRβ rat monoclonal antibody CL10; and
an antibody or a fragment thereof, comprising a sequence having a substitution of serine 126 of SEQ ID NO: 34 (the nucleotide sequence: tct) with cysteine (Cys; the nucleotide sequence: tgt) in the amino acid sequence of the light chain variable region (VL) (SEQ ID NO: 34; the corresponding nucleotide sequence: SEQ ID NO: 35) and the amino acid sequence of the heavy chain variable region (VH) (SEQ ID NO: 36; the corresponding nucleotide sequence: SEQ ID NO: 37), derived from the anti-mouse FRβ rat monoclonal antibody CL10.

A humanized antibody comprises the sequences of the heavy chain CDR1 to CDR3 and the light chain CDR1 to CDR3 of the above antibody and the sequences of the heavy chain framework region and constant region and the light chain framework region and constant region, derived from a human, for example. The amino acid sequences of the framework region and the constant region can comprise mutation, such as substitution, deletion, or addition (or insertion) of 1 to 3, and preferably 1 or 2 amino acid residues, without changing the binding specificity for the FRβ protein. Specific examples of humanized antibodies are provided below:
a humanized antibody or a fragment thereof, comprising the light chain variable region (VL) comprising CDR1, CDRL2, and CDRL3 of the amino acid sequences as shown in SEQ ID NOs: 8, 9, and 10, respectively, and the heavy chain variable region (VH) comprising CDR1, CDRH2, and CDRH3 of the amino acid sequences as shown in SEQ ID NO: 11, SEQ ID NO: 12, and SEQ ID NO: 13, respectively;
a humanized antibody or a fragment thereof, comprising the light chain variable region (VL) comprising CDR1, CDRL2, and CDRL3 of amino acid sequences as shown in SEQ ID NOs: 18, NO: 19, and NO: 20, respectively, and the heavy chain variable region (VH) comprising CDR1, CDRH2, and CDRH3 of the amino acid sequences as shown in SEQ ID NO: 21, SEQ ID NO: 22, SEQ ID NO: 23, respectively;
a humanized antibody or a fragment thereof, comprising the light chain variable region (VL) comprising CDR1, CDRL2, and CDRL3 of the amino acid sequences as shown in SEQ ID NOs: 28, 29, and 30, respectively, and the heavy chain variable region (VH) comprising CDR1, CDRH2, and CDRH3 of the amino acid sequences as shown in SEQ ID NOs: 31, 32, and 33, respectively; and
a humanized antibody or a fragment thereof, comprising the light chain variable region (VL) comprising CDR1, CDRL2, and CDRL3 of the amino acid sequences as shown in SEQ ID NOs: 38, 39, and 40, respectively, and the heavy chain variable region (VH) comprising CDR1, CDRH2, and CDRH3 of the amino acid sequences as shown in SEQ ID NOs: 41, 42, and 43, respectively.

As described in the examples below, after mutant VH and mutant VL were prepared by introducing Cys substitution into VH or VL of the antibody 94b or VH or VL of the antibody CL10, a toxin polypeptide (e.g., Pseudomonas Exotoxin (PE)) is allowed to fuse to each mutant VH to prepare an immunotoxin VH, to which each mutant VL is subsequently allowed to bind, thereby producing an immunotoxin. For example, the immunotoxin comprises the amino acid sequence of the anti-human FRβ immunotoxin VHPE as shown in SEQ ID NO: 44 and the amino acid sequence of the mutant VL as shown in SEQ ID NO: 45. Alternatively, the immunotoxin comprises the amino acid sequence of the anti-mouse FRβ immunotoxin VHPE as shown in SEQ ID NO: 46 and the amino acid sequence of the mutant VL as shown in SEQ ID NO: 47.

The antibodies of the present invention bind immunologically and specifically to cell-surface FRβ proteins of FRβ macrophages located around pancreatic cancer cells at the invasive front, and the dissociation constant (K_{d}) is, for example, 1 x 10⁻⁷ M or less, 1 x 10⁻⁸ M or less, 1 x 10⁻⁹ M or less, 1 x 10⁻¹⁰ M or less, 1 x 10⁻¹¹ M or less, 1 x 10⁻¹² M or less, 1 x 10⁻¹³ M or less, 1 x 10⁻¹⁴ M or less, or 1 x 10⁻¹⁵ M or less.

### <Molecular-targeted anticancer agent>

According to the present invention, a molecular-targeted anticancer agent comprising the antibodies and a toxin or cytotoxic agent bound thereto is used as an active ingredient of a therapeutic agent for invasive pancreatic cancer or for the production of such therapeutic agent.

The toxin or cytotoxic agent has the capacity for killing or damaging FRβ macrophages located around pancreatic cancer cells at the invasive front. This can inhibit the growth and/or metastasis of pancreatic cancer.

Examples of toxins include, but are not limited to, bacterial toxins, phytotoxins, endotoxins, and exotoxins. Specific examples include bacterial toxins, such as diphteria toxin, Pseudomonas toxin, ricin A chain or deglycosylated ricin A chain of Pseudomonas aeruginosa exotoxin, Pseudomonas exotoxin PE38, ribosome inactivating protein, abrin A chain, modeccin A chain, alpha-sarcin, gelonin, aspergillin, restrictocin, ribonuclease, epidophyllotoxin, diphtheria toxin, diphtheria A chain, and staphylococcus enterotoxin.

Examples of cytotoxic agents against tumor cells include antitumor agents, tumor growth inhibitors, cell cycle arrest inducers, DNA synthesis inhibitors, transcription inhibitors, translation/protein synthesis inhibitors, cell division inhibitors, inducers of apoptosis against tumor cells, and radioactive nuclides.

Examples of cytotoxic agents include, but are not limited to, pokeweed anti-viral protein, abrin, ricin and ricin A chain, altretamine, actinomycin D, plicamycin, puromycin, gramicidin D, doxorubicin, colchicine, cytochalasin B, cyclophosphamide, emetine, maytansine, amsacrine, cisplatin, etoposide, etoposide ortho-quinone, teniposide, daunorubicin, gemcitabine, doxorubicin, mitoxantraone, bisantrene, bleomycin, methotrexate, vindesine, vinorelbine, podophyllotoxin, adriamycin, vincristine, vinblastine, BCNU, Taxol, Tarceva, Avastin, mitomycin, modified Pseudomonous enterotoxin A, calicheamicin, 5-fluorouracil, cyclophosphamide, and cytokines such as TNF-α and NF-β.

Examples of radioactive nuclides include, but are not limited to, radioactive isotopes, such as lead-212, bismuth-212, astatine-211, iodine-131, scandium-47, rhenium-186, rhenium-188, samarium-153, yttrium-90, iodine-123, iodine-125, iodine-131, bromine-77, indium-111, phosphorus-32, and boron-10, or fissionable nuclides such as actinides. Labeling can be performed via an amino acid residue in a protein, such as a cysteine or lysine residue. Labeling techniques are described in, for example, Monoclonal Antibodies in Immunoscintigraphy (Chatal, CRC Press, 1989).

When the molecular-targeted anticancer agent of the present invention comprises the antibody and the toxin (i.e., an immunotoxin), such ingredients can be in the form of a fusion protein. In such a case, a toxin can bind to, for example, the framework region or the C-terminal region in the variable region or the CH3 region or the C-terminal region in the constant region of the antibody protein through a linker (e.g., a peptide), according to need. When the molecular-targeted anticancer agent of the present invention comprises the antibody and the cytotoxic agent, however, such ingredients can bind to each other via a covalent or non-covalent bond through functional groups. For example, a reactive group in an antibody molecule, such as a functional group such as an amino, carboxyl, hydroxyl, or mercapto group, is allowed to react with a toxin or cytotoxic agent having a reactive group, so as to produce a molecular-targeted agent. Examples of the reactive group include N-succinimidyl ester, N-sulfosuccinimidyl ester, carboxyl, amino, mercapto, and disulfide groups. Binding can be performed with the use of a coupling agent, such as a bifunctional coupling agent, active ester, aldehydes, bisazide, or isocyanate.

### <Pharmaceutical composition>

The present invention also provides a pharmaceutical composition for inhibiting growth and/or metastasis of invasive pancreatic cancer in a subject, which comprises the molecular-targeted anticancer agent in combination with a pharmaceutically acceptable carrier.

The molecular-targeted anticancer agent of the present invention is capable of inhibiting the metastasis, in particular, lymph node metastasis or hematogenous metastasis, in addition to growth inhibition. Through inhibition of such metastasis, neoplasm metastasis to organs other than the pancreas can be inhibited. Since the molecular-targeted agent of the present invention binds specifically to the FRβ (+) macrophages existing at the invasive front of invasive pancreatic cancer, pancreatic cancer in the vicinity thereof can be selectively attacked, and the influence imposed on normal cells can be minimized.

A pharmaceutically acceptable carrier (or an excipient) may be liquid or solid, and an adequate carrier can be selected in accordance with a type of an oral or parenteral preparation. Examples thereof include sterile water, buffer such as PBS, physiological saline, Ringer's solution, ethanol, glycerol, vegetable oil, gelatin, sucrose, lactose, amylose, starch, fatty acid ester, and hydroxymethylcellulose. In addition to such carrier, the pharmaceutical composition can further comprise an adjuvant, such as a lubricant, preservative, stabilizer, wetting agent, emulsifier, disintegrator, solubilizer, isotonizing agent, binder, buffer, or colorant, according to need.

The pharmaceutical composition of the present invention can be administered orally, intravenously, intraarterially, transmucosally, intramuscularly, subcutaneously, buccally, intraperitoneally, intraarticularly, intrasynovially, sternally, intranasally, or via bolus injection, continuous injection, or direct delivery to the lesion.

The pharmaceutical composition is formulated in accordance with the route of administration. Examples of dosage forms include, but are not particularly limited to, injection preparations, solutions, suspensions, tablets, granules, powders, sprayers, capsules, enteric preparations, controlled-release agents, and multiple-layer preparations.

The molecular-targeted anticancer agent, which is an active ingredient of the pharmaceutical composition of the present invention, is integrated into a unit dosage form in a therapeutically effective amount. A dose can be altered in accordance with a variety of factors, such as sexuality, age, and body weight of a subject, the severity, the route of administration, and side effects. The amount of active ingredients is about 1 µg or more per day (e.g., at least 50 µg to 100 µg), although the amount is not limited thereto. Administration may be either a single administration or multiple administrations.

The pharmaceutical composition of the present invention can be administered in combination with a conventional therapeutic agent for pancreatic cancer. Examples of conventional therapeutic agents include pharmaceuticals, such as gemcitabine, 5-FU, cisplatin, Gemzar, and TS-1. Such therapeutic agents can be administered to a subject, prior to, simultaneously with, or following the administration of the pharmaceutical composition of the present invention.

### <Methods for detecting the degree of malignancy of pancreatic cancer or the presence of invasive pancreatic cancer and diagnostic agents and kits used for such methods>

Another aspect of the present invention provides a method for detecting the degree of malignancy of pancreatic cancer or the presence of invasive pancreatic cancer comprising bringing a pancreatic cancer tissue sample obtained from the subject into contact with an antibody that specifically binds to the cell-surface FRβ protein of the FRβ (+) macrophages (an antibody labeled with a radioactive isotope, fluorophore, or pigment or a non-labeled antibody), examining the presence or absence of the FRβ (+) macrophages in the vicinity of the pancreatic tissue at the invasive front of the cancer tissue based on the formation of a conjugate of the FRβ protein and the antibody or a fragment thereof, and determining that the cancer tissue is invasive and metastatic when the FRβ (+) macrophages are distributed around pancreatic cancer cells at the invasive front.

The term "determine" used herein does not intend to refer to a judgment made by a doctor (i.e., medical act), but the term refers to a means for assisting a doctor in making decisions through presentation of the information or data concerning the examination results to a doctor. Accordingly, the term "determine" should be used interchangeably with the term, such as "measure," "examine," "judge," or "evaluate."

In addition, the present invention provides a diagnostic agent or kit comprising the antibody, or a fragment thereof, used for the above detection method, which is intended to diagnose the degree of malignancy of pancreatic cancer or the presence of invasive pancreatic cancer (including imaging diagnosis).

In the case of imaging diagnosis, the diagnostic agent or kit can comprise a conjugate of the antibody or a fragment thereof and a label. Examples of labels include a fluorophore, a pigment, and a radioactive isotope as exemplified above.

Pancreatic tissue samples are obtained from subjects suspected to suffer from pancreatic cancer via surgery.

The term "invasion" or "infiltration" as used herein refers to the situation in which the tumor departs from the deep part of the lesion tissue, and from the primary lesion, as tumor cell motility is enhanced. When a tumor is determined to be invasive, the tumor has become metastatic and malignant.

The method of the present invention is based on the correlation between the cancer front becoming invasive and FRβ (+) macrophages accumulating. By detecting the presence of FRβ (+) macrophages with the use of the antibody or a fragment thereof, the pancreatic cancer of interest is determined (judged, identified, classified, or evaluated) to be invasive and metastatic.

Detection of FRβ macrophages (+) with the use of antibodies can be performed via, for example, ELISA, fluorescent antibody methods, radioimmunodetection, sandwich methods, or histological tissue staining. Labels, such as enzymes (e.g., peroxidase or alkaline phosphatase), fluorophores (e.g., FITC, tetramethylrhodamine, or Texas Red), pigments, or radioactive isotopes, are conjugated to secondary antibodies, and conjugates of antibodies bound to macrophages are detected. Examples of label binding include chemical binding and biotin-(strepto)avidin-based binding.

In the case of the imaging diagnosis, antibodies are labeled with pharmaceutically acceptable radioactive nuclides or luminophores, the resulting antibodies are administered to a subject, images are generated through diagnostic imaging techniques, such as PET/CT, and the presence of pancreatic cancer can be determined or detected.

The kit of the present invention can comprise, in addition to the (labeled or non-labeled) antibodies mentioned above or fragments thereof and contrast media comprising the same, a buffer used for measurement, reagents (e.g., labeled secondary antibodies), instructions for measurement procedures, and the like. Reagents are hermetically sealed in separate containers.

### Examples

Hereafter, the present invention is described in greater detail with reference to the examples, although the technical scope of the present invention is not limited thereto.

### [Example 1]

### Production of anti-human FRβ mouse monoclonal antibody and anti-mouse FRβ rat monoclonal antibody

### [Preparation of cells expressing FRβ as antigen]

Total RNA was extracted from the articular rheumatism synovial membrane or Balb/c mouse liver using Trizol (GibcoBRL) and the cDNA synthesis kit (Invitrogen) in accordance with the manufacturer's instructions, and cDNA was then synthesized using the SuperScript plasmid System (Invitrogen) in accordance with the manufacturer's instructions. Subsequently, 1 µl cDNA derived from the rheumatism synovial membrane or Balb/c mouse liver was separately added to the Bioneer PCR premix (Bioneer), the sense primer (the human rheumatism synovial membrane: agaaagacatgggtctggaaatggatg (SEQ ID NO: 48) or the mouse liver: tctagaaagacatggcctggaaacag (SEQ ID NO: 49)) and the antisense primer (the human rheumatism synovial membrane: gactgaactcagccaaggagccagagtt (SEQ ID NO: 50) or the mouse liver: cccaacatggatcaggaact (SEQ ID NO: 51)) adjusted to 10 pmol were added, and PCR was carried out through 30 cycles of 94°C for 20 seconds, 58°C for 30 seconds, and 72°C for 60 seconds, followed by the reaction at 72°C for 5 minutes, to amplify the human or mouse FRβ. The PCR product of the amplified FRβ gene was ligated to the PCR2.1-TOPO plasmid (Invitrogen). Specifically, 1 µl of a NaCl solution, 1.5 µl of sterile distilled water, and 1 µl of the plasmid vector (PCR2.1-TOPO) were added to 2.5 µl of the PCR product, the mixture was incubated at room temperature for 5 minutes, 2 µl of the product was added to E. coli cells (TOP10F'), the resultant was subjected to the reaction on ice for 30 minutes, it was subjected to thermal treatment at 42°C for 30 seconds, the resultant was allowed to stand on ice for 2 minutes, 250 µl of SOC medium was added thereto, and the resultant was cultured in a shaker at 37°C for 1 hour. After the completion of culture, the culture product was seeded on an LB medium, and culture was conducted at 37°C overnight.

In order to culture E. coli cells, white colonies sampled from the plate were applied to LB liquid medium containing 50 µg/ml ampicillin to conduct culture at 37°C overnight. Plasmids in E. coli cells were purified using the Qiagen plasmid purification kit (Qiagen). The integrated FRβ gene was treated with the restriction enzyme EcoRI and then developed into agarose electrophoresis. After the FRβ gene product of about 0.8 kb (782 bp) was detected, the site was cut out, and the extracted gene product was purified using the Qiagen PCR purification kit (Qiagen). Subsequently, the resultant was mixed with the mammalian cell expression vector, which had been treated with EcoRI in advance (pER-BOS, Mizushima et al. pEF-BOS, a powerful mammalian expression vector, Nucleic Acid Res. 1990; 18 (17): 5322), and ligation was carried out using the T4 ligase (Roche). The ligation product was transduced into the E. coli cells (TOP10F') and the FRβ gene was detected in the manner described above.

After the FRβ gene integrated into pEF-BOS was confirmed, a vector comprising human FRβ was transduced into the mouse B300-19 cells, and a vector comprising mouse FRβ was transduced into the rat RBL2H3 cells. Specifically, a mixture of 20 µl of lipofectamine (GibcoBRL) with 1 µg of the FRβ vector was each added to each of the cells adjusted to a density of 1 x 10⁵ cells. In order to acquire G418-tolerance, the transduced mouse B300-19 cells and rat RBL2H3 cells were subjected to selective culture in a medium containing G418 at 1 mg/ml. Introduction of the FRβ gene into the transduced cells was confirmed via PCR. Specifically, cDNA was synthesized from the adjusted 1x10⁷ cells using the cDNA synthesis kit (Invitrogen), the sense primer (the human rheumatism synovial membrane: agaaagacatgggtctggaaatggatg (SEQ ID NO: 48) or the mouse liver: tctagaaagacatggcctggaaacag (SEQ ID NO: 49)) and the antisense primer (the human rheumatism synovial membrane: gactgaactcagccaaggagccagagtt (SEQ ID NO: 50) or the mouse liver: cccaacatggatcaggaact (SEQ ID NO: 51)) adjusted to 10 pmol were added to the Bioneer PCR premix (Bioneer). PCR was carried out through 30 cycles of 94°C for 20 seconds, 58°C for 30 seconds, and 72°C for 60 seconds, followed by the reaction at 72°C for 5 minutes, to amplify the human or mouse FRβ. Thereafter, agarose electrophoresis was carried out to confirm a 0.8-kb band indicated by FRβ.

### [Production of anti-human FRβ mouse monoclonal antibody and anti-mouse FRβ rat monoclonal antibody]

The FRβ-expressing mouse B300-19 cells or rat RBL2H3 cells were adjusted at 1 x 10⁷ cells, the cells were mixed with the Freund's complete adjuvant, and Balb/c mice (for the anti-human FRβ monoclonal antibodies) or Whister Kyoto rats (for the anti-mouse FRβ monoclonal antibodies) were immunized therewith through the tail heads or intraperitoneally. This procedure was repeated 2 to 4 times every week.

Monoclonal antibodies were produced by the Koler's method (Kohler & Milstein, Nature, 1975, 256: 495-96). Specifically, the spleens or iliac lymph nodes were removed and dissociated into single cells, and the resultant was fused to myeloma-derived cells (NS-1) to prepare hybridomas. Hybridomas were cultured in a HAT selection medium, and antibodies secreted into the culture supernatant were selected based on the reactivity with the FRβ-expressing cells.

Hybridomas that were found to produce antibodies were subjected to cloning by limiting dilution culture diluted to 1 cell/well in a 96-well plate.

Antibodies produced by the cloned hybridomas were obtained from the mouse ascites. At the outset, the cell count was adjusted to 1 x 10⁷ cells, the cells were administered intraperitoneally to nude mice, and the mice were allowed to grow until peritoneal cavity expansion was observed. Ascites was obtained from the mice in which peritoneal cavity expansion was observed, and monoclonal antibodies were purified therefrom using the Protein G column (GE BioSciences). The isotypes of the purified mouse and rat monoclonal antibodies were determined using the isotyping ELISA kit (Pharmingen). As a result, Clone 36b of type IgG2a and Clone 94b of type IgG1 of the anti-human FRβ mouse monoclonal antibodies were obtained, while Clones CL5 and CL10 of type IgG2a of the anti-mouse FRβ rat monoclonal antibodies were obtained. The reactivity of such antibodies to antigens was analyzed via flow cytometry.

### [Determination of the heavy chain variable regions (VHs), the light chain variable regions (VLs), the VH sequences, and the VL sequences of the anti-human FRβ mouse monoclonal antibodies and the anti-mouse FRβ rat monoclonal antibodies]

Mouse hybridoma clones 36b and 94b were adjusted to 1 x 10⁷ cells, and cDNA was synthesized using the cDNA synthesis kit (Invitrogen). The VH and VL genes of Clones 36b and 94b were determined via PCR using the Ig-Prime Kit. PCR was carried out in accordance with the instructions. Specifically, a cycle of 94°C for 60 seconds, 50°C for 60 seconds, and 72°C for 120 seconds was repeated for 30 cycles, followed by the reaction at 72°C for 5 minutes, to amplify the VH and VL genes. The VH and VL products amplified by PCR were ligated to the PCR2.1-TOPO plasmid (Invitrogen) and transduced into E. coli (TOP10F'). Plasmids were purified from the transduced E. coli, and the nucleotide sequences of VH and VL of Clones 36b and 94b were determined. The nucleotide sequences were determined by performing PCR using the BigDye Terminaor V3.1 cycle sequencing kit (ABI) and analyzed using the ABI 310 DNA sequencer (VL of 36b (SEQ ID NO: 5), VH of 36b (SEQ ID NO: 7), VL of 94b (SEQ ID NO: 15), and VH of 94b (SEQ ID NO: 17)).

Rat hybridoma clones CL5 and CL10 were adjusted to 1 x 10⁷ cells, and cDNA was synthesized using the cDNA synthesis kit (Invitrogen). Subsequently, the VH and VL genes were amplified via PCR using the Ig-Prime Kit and the primer (caccatggagttacttttgag (SEQ ID NO: 52)) designed for rat VH amplification. The VH and VL products amplified via PCR were ligated to the PCR2.1-TOPO plasmid (Invitrogen) and the resultant was transduced into E. coli (TOP10F'). Subsequently, plasmids were purified from E. coli, and the nucleotide sequences of VH and VL were determined. The nucleotide sequences were determined by performing PCR using the BigDye Terminaor V3.1 cycle sequencing kit (ABI) and analyzed using the ABI 310 DNA sequencer (VL of CL5 (SEQ ID NO: 25), VH of CL5 (SEQ ID NO: 27), VL of CL10 (SEQ ID NO: 35), and VH of CL10 (SEQ ID NO: 37)).

### [Example 2]

### Production of recombinant immunotoxin (i.e., molecular-targeted agent)

### [Introduction of cysteine mutation into variable region of immunoglobulin heavy chain gene (VH)]

Primers were designed so as to cause mutation of glycine (the nucleotide sequence: ggc) at amino acid 63 in the variable region of the immunoglobulin heavy chain gene of the anti-human FRβ mouse monoclonal antibody 94b (VH, SEQ ID NO: 16) with cysteine (the nucleotide sequence: tgt) (sense primer: cagaggcctgaacattgtctggagtggattggaag (SEQ ID NO: 53); antisense primer: cttccaatccactccagacactgttcaggcctctg (SEQ ID NO: 54)), and the pCR2.1-TOPO 94bVH plasmid comprising a 94b VH obtained in Example 1 was subjected to mutagenesis using the Quick change site-directed mutagenesis kit (Stratagene). PCR was carried out through 12 continuous cycles of 95°C for 30 seconds, 55°C for 60 seconds, and 68°C for 4 minutes. The cysteine codon was introduced into the variable region of the immunoglobulin heavy chain gene of the anti-mouse FRβ rat monoclonal antibody CL10 (VH, SEQ ID NO: 36) with the use of the primers designed in the manner as described above (sense primer: gtccgccaggctccaacgaagtgtctggagtgg gtcgc (SEQ ID NO: 55); antisense primer: gcgacccactccagacacttcgttggagcctggcggac (SEQ ID NO: 56)).

Subsequently, DNA was transduced into E. coli XL1-Blue after the reaction, and selective culture was conducted in an LB medium containing ampicillin at 0.1 mg/ml. Plasmids of the selected transformants were purified using the QIAprep spin Miniprep Kit (Qiagen). Further, the nucleotide sequence was determined using the BigDye Terminaor V3.1 cycle sequencing kit (ABI) and the ABI 310 DNA sequencer, and mutation of glycine 63 with cysteine (the nucleotide sequence: tgt) was confirmed.

### [Insertion of transduced VH into pRK79PE38 vector]

Subsequently, VH comprising mutations of 94bVH and CL10VH was inserted into the pRK79 vector (pRK79PE38) containing the PE38 gene in the manner described below.

For annealing of the 5' end and the 3' end of the transduced 94b, the primers taagaaggagatatacatatggaggttcagctgcagcagtc (SEQ ID NO: 57) and gccctcgggacctccggaagcttttgaggagactgtgagagtgg (SEQ ID NO: 58) were designed, and the primers atacatatggaggtgcagctggtggagtctggg (SEQ ID NO: 59) and tccggaagcttttgaggagacagtgactgaagc (SEQ ID NO: 60) were designed for annealing of the 5' end and the 3' end of CL10. One of the set of primers for annealing comprises the NdeI restriction enzyme, and cloning at this site enables protein expression using atg as an initiation codon. The HindIII site has been inserted into another primer, and cloning at this site enables expression of a fusion protein of VH and the PE gene.

The pCR2.1-TOPO-94bVH and pCR2.1-TOPO-CL10VH plasmids into which mutation had been introduced with the use of such set of primers and pfu DNA polymerase (Stratagene) were subjected to PCR. PCR was carried out through 30 cycles of 94°C for 20 seconds, 55°C for 30 seconds, and 72°C for 60 seconds, followed by the reaction at 72°C for 5 minutes. Subsequently, the PCR product was purified, the NdeI and HindIII (New England Biolabs) restriction enzymes were added to the purified product, the resultant was subjected to electrophoresis, and DNA of the target size was recovered from the gel using the QIAquick gel extraction kit (Qiagen). To the recovered DNA, pRK79PE38 treated with the same restriction enzymes as the transduced VH treated with the restriction enzymes was added, and VH and pRK79PE38 were subjected to ligation using Ligation High (Toyobo). After the completion of ligation, E. coli TOP10F' (Invitrogen) was transduced, and transformants were selected in an LB medium containing ampicillin at 0.1 mg/ml. The pRK79-VHPE plasmids of the selected transformants were purified using the QIAprep spin Miniprep Kit (Qiagen). Further, the nucleotide sequence was determined using the BigDye Terminaor V3.1 cycle sequencing kit (ABI) and the ABI 310 DNA sequencer (VHPE of anti-human FRβ immunotoxin (SEQ ID NO: 44); and VHPE of anti-mouse FRβ immunotoxin (SEQ ID NO: 46)).

### [Introduction of cysteine mutation into variable region of immunoglobulin light chain gene]

Primers were designed so as to cause mutation of amino acid 125 in the variable region of the immunoglobulin light chain gene of the anti-human FRβ mouse monoclonal antibody 94b (VL, SEQ ID NO: 15) with cysteine (the nucleotide sequence: tgt) (sense primer: taagaaggagatatacatatggacattgtgatgtcacaatc (SEQ ID NO: 61); this primer comprises nucleotides (catatg) that can be cleaved with the NdeI restriction enzyme, and cloning at this site accordingly enables protein expression using atg as the initiation codon); and antisense primer: gctttgttagcagccgaattcctatttgatttccagcttggtgccacaaccgaacgt (SEQ ID NO: 62); this primer is designed to cause mutation of amino acid 125 with cysteine (tgt) and to comprise nucleotides (gaattc) that can be cleaved with the EcoRI restriction enzyme, following the termination codon (tag)). Also, primers were designed so as to cause mutation of amino acid 126 in the variable region of the immunoglobulin light chain gene of the anti-mouse FRβ rat monoclonal antibody CL10 (VL, SEQ ID NO: 34) with cysteine (the nucleotide sequence: tgt) (atacatatggacattgtgatgcccaatctccatcc (SEQ ID NO: 63) and gctttgttagcagccgaattcctatttgatttccagcttggtgccacaaccgaacgt (SEQ ID NO: 62)).

The pCR2.1-TOPO-94bVL plasmid was subjected to PCR with the use of such set of primers and pfu DNA polymerase (Stratagene). PCR was carried out through 30 cycles of 94°C for 20 seconds, 55°C for 30 seconds, and 72°C for 60 seconds, followed by the reaction at 72°C for 5 minutes. Subsequently, the PCR product was purified, the NdeI and EcoRI (New England Biolabs) restriction enzymes were added to the purified product, the resultant was subjected to electrophoresis, and DNA of the target size was recovered from the gel using the QIAquick gel extraction kit (Qiagen). To the recovered DNA, pRK79PE38 treated with the same restriction enzymes as the transduced VL treated with the restriction enzymes was added, and VH and pRK79PE38 were subjected to ligation using Ligation High (Toyobo). After the completion of ligation, E. coli TOP10F' (Invitrogen) was transduced, and transformants were selected in an LB medium containing ampicillin at 0.1 mg/ml. The pRK79-VLPE plasmids of the selected transformants were purified using the QIAprep spin Miniprep Kit (Qiagen). Further, the nucleotide sequence was determined using the BigDye Terminaor V3.1 cycle sequencing kit (ABI) and the ABI 310 DNA sequencer, and mutation of amino acid 126 of the transduced VL with cysteine and the provision of the termination codon (tag) were confirmed (VL of anti-human FRβ immunotoxin (SEQ ID NO: 45); and VL of anti-mouse FRβ immunotoxin (SEQ ID NO: 47)).

### [Preparation of recombinant protein inclusion body]

The pRK79-94bVHPE and pRK79-CL10VHPE plasmids into which the transduced VH had been integrated and the pRK79-VL94b and pRK79-VLCL10 plasmids into which the transduced VL had been integrated (50 ng each) were prepared, and the plasmids were transduced into protein-expressing E. coli BL21 (DE3). The transduced E. coli was selected through culture in an LB medium containing ampicillin at 0.1 mg/ml at 37°C for 15 to 18 hours.

After the completion of selection, E. coli was cultured in 1,000 ml Super broth medium at 37°C, and culture was continued until the visible-light absorption at 600 nm reached 1.0 to 1.5. Thereafter, IPTG (isopropyl-beta-D-thio-galactopyranoside) was added to the medium to result in the final concentration of 1 mM, and culture was further conducted at 37°C for 90 minutes. After the completion of culture, E. coli cells were recovered via centrifugation, and 200 ml of a suspension thereof was prepared with the use of 50 mM Tris buffer (pH 7.4, containing 2.5% Triton X-100, 0.5 M NaCl, and 20 mM EDTA). Thereafter, egg white lysozyme was added to a final concentration of 0.2 mg/ml, the reaction was allowed to proceed at room temperature for 1 hour to destroy E. coli cells. Thereafter, the resultants were centrifuged at 20,000 x g, and the precipitate was recovered. The precipitate was further suspended in 50 mM Tris buffer (pH 7.4, containing 2.5% Triton X-100, 0.5 M NaCl, and 20 mM EDTA) to adjust the amount of the solution to 200 ml, egg white lysozyme was added to a final concentration of 0.2 mg/ml, and the reaction was allowed to proceed at room temperature for 1 hour. After the completion of the reaction, the reaction product was centrifuged at 20,000 x g, and the precipitate was recovered. Thereafter, the precipitate was suspended to 200 ml with 50 mM Tris buffer (pH 7.4, containing 2.5% Triton X-100, 0.5 M NaCl, and 20 mM EDTA), and then was thoroughly mixed and centrifuged at 20,000 x g, and the precipitates were then recovered. This procedure was repeated 5 times, and the resulting precipitate, which was a recombinant immunotoxin inclusion body, was further dissolved in 0.1M Tris buffer (pH8.0, containing 6 M guanidine hydrochloride and 1 mM EDTA) to adjust it to the final concentration to 10 mg/ml.

### [Coupling of solubilized VHPE and VL, refolding, and purification]

94b-VHPE was mixed with 94b-VL and CL10-VHPE was mixed with CL10-VL to produce the recombinant double-chain Fv anti-FRβ immunotoxin.

At the outset, 0.5 ml of VHPE was admixed with 0.25 ml of VL, dithiothreitol (DTT) was added to a final concentration of 10 mg/ml, and the resultant was subjected to reduction at room temperature for 4 hours. After reduction, the resultant was dissolved in 75 ml of 0.1 M Tris buffer (pH 8.0, containing 0.5 M arginine, 0.9 mM oxidized glutathione, and 2mM EDTA). The resulting solution was allowed to stand at 10°C for 40 hours to bind VH to VL. After the completion of binding, the solution was concentrated to the amount of 5 ml with the use of a centrifugal concentrator with the molecular-weight cut-off 10,000 (Centricon 10, Amicon), and the concentrate was then diluted with 50 ml of Tris buffer (pH 7.4, containing 0.1 M urea and 1 mM EDTA). The diluted solution was used as a starting material for purification of the recombinant immunotoxin.

Subsequently, the starting material was allowed to adsorb to an ion-exchange column (Hi-Trap Q, GE) equilibrated with Tris buffer (pH 7.4, containing 1 mM EDTA) at the flow rate of 30 ml/hr, followed by washing with Tris buffer (pH 7.4, containing 1 mM EDTA). Thereafter, the adsorbed recombinant immunotoxin was eluted with Tris buffer (pH 7.4, containing 0.3 M NaCl and 1 mM EDTA). The eluted sample was dialyzed against Tris buffer (pH 7.4, 1 containing mM EDTA) and further purified through an ion-exchange column (POROS HQ, POROS). Specifically, the dialyzed and purified substance was allowed to adsorb to the column at the flow rate of 10 ml/min, the column was washed with Tris buffer (pH 7.4, containing 1 mM EDTA), and elution of the recombinant immunotoxin was carried out with a NaCl gradient of 0 M to 1.0 M in the buffer. The final product of the purified recombinant immunotoxin was prepared via gel filtration chromatography (TSK300SW, Tosoh). At the outset, endotoxin was removed from the TSK300SW column by washing with 75% ethanol for disinfection for 48 hours. Subsequently, the TSK300SW column was washed with distilled water for injection according to the Japanese Pharmacopoeia and then equilibrated with physiological saline according to the Japanese Pharmacopoeia. After the completion of equilibration, the recombinant immunotoxin was administered, and the eluate from the column was obtained at the flow rate of 0.25 ml/ min. Thereafter, the eluate was applied to a 0.22-µm filter sterilizer, the purity was determined by SDS-PAGE, and the resultant was then stored at -80°C.

### [Purity determined by SDS-PAGE]

SDS-PAGE (electrophoresis on polyacrylamide gel containing sodium dodecyl sulfate) was carried out using a 12% polyacrylamide flat gel containing 0.1 % sodium dodecyl sulfate (SDS) and, as a mobile phase, an aqueous solution comprising SDS (final concentration: 0.1%), 130 mM glycine, and 25 mM Tris. The sample was adjusted with 100 mM Tris buffer (pH 6.5) containing SDS (final concentration: 0.1%) and subjected to boiling for 5 minutes. After the completion of boiling, the sample was applied to the flat gel and electrophoresis was carried out at the constant current of 30 mA. Thereafter, the recombinant immunotoxin was stained with a solution of 0.05% Coomassie brilliant blue R (Nacalai Tesque).

### [Example 3]

### Expression of macrophages in human pancreatic cancer tissue

While CD68-expressing macrophages (i.e., invasive macrophages) were observed both the inside and the front of pancreatic cancer, CD163-expressing macrophages were expressed at the tumor front, FRβ macrophages were also expressed at the tumor front (Fig. 3), and FRβ (+) macrophages were also co-expressed with CD68 (Fig. 4). However, the number of FRβ-positive macrophages invading the pancreatic cancer was found to be smaller than that of CD68-positive macrophages at a significant level (Fig. 5).

### [Example 4]

### Detection of FRβ macrophage at pancreatic cancer front using anti-mouse FRβ rat monoclonal antibody

The human pancreatic cancer cell line (Capan-1) was transplanted into a nude mouse, and the transplanted tumor was stained with the mouse FRβ antibody for comparison. Based on comparison between the center of the transplanted tumor (Figs. 6A and 6C) and the peripheral zone thereof (Figs. 6B and 6D), FRβ (+) macrophages were found to be expressed in a nude mouse and to be distributed at the tumor front.

### [Example 5]

### Detection of FRβ (+) macrophages in human pancreatic cancer and correlation thereof with metastasis

The human pancreatic cancer tissue samples collected from subjects via surgery (n=76) were examined for FRβ (+) macrophage expression, lymphatic metastasis, and hematogenous metastasis. As a result, FRβ expression in FRβ (+) macrophages was found to be highly correlated with lymphatic and hematogenous metastasis (Table 1). FRβ (+) macrophages were found to be distributed around newborn blood vessels at the tumor front (Fig. 7A, 7B, 7C). In addition, the correlation was observed between high FRβ expression by FRβ (+) macrophages and high density of the new tumor blood vessels (Fig. 8). In addition, it was found that hematogenous metastasis would occur at high frequency in the group of subjects exhibiting high FRβ expression by FRβ (+) macrophages (Fig. 9).

**Table 1: Correlation between the number of CD68-, CD163-, or FRβ-positive cells at the invasive front of human pancreatic adenocarcinoma and clinopathological factors (n = 76)**

| | | CD68⁺ cells | | | CD163⁺ cells | | | FRβ⁺ cells | | |
|---|---|---|---|---|---|---|---|---|---|---|
| Characteristics (n) | | Low | High | P | Low | High | P | Low | High | P |
| Sex | | | | | | | | | | |
| | Male (52) | 28 | 24 | 0.516 | 25 | 27 | 0.622 | 24 | 28 | 0.323 |
| | Female(24) | 11 | 13 | | 13 | 11 | | 14 | 10 | |

| Histological finding | | | | | | | | | | |
|---|---|---|---|---|---|---|---|---|---|---|
| | Good (30) | 17 | 13 | 0.451 | 19 | 11 | 0.059 | 16 | 14 | 0.639 |
| | Moderate/poor (46) | 22 | 24 | | 19 | 27 | | 22 | 24 | |

| Lymph node metastasis | | | | | | | | | | |
|---|---|---|---|---|---|---|---|---|---|---|
| | Negative (29) | 17 | 12 | 0.316 | 21 | 8 | 0.002 | 20 | 9 | 0.009 |
| | Positive (47) | 22 | 25 | | 17 | 30 | | 18 | 29 | |

| Hematogenous metastasis | | | | | | | | | | |
|---|---|---|---|---|---|---|---|---|---|---|
| | Negative (49) | 25 | 24 | 0.945 | 28 | 21 | 0.092 | 31 | 18 | 0.002 |
| | Positive (27) | 14 | 13 | | 10 | 17 | | 7 | 20 | |

| | | | | | | | | | | |
|---|---|---|---|---|---|---|---|---|---|---|
| P values were calculated by the X² test. | | | | | | | | | | |

### [Example 6]

### Relationship of FRβ (+) macrophage expression and survival time in human pancreatic cancer

Surgical cases (n=76) of human pancreatic cancer were used to examine for FRβ (+) macrophage expression and survival time. While CD68-positive macrophage showed no significant differences between the FRβ (+) macrophage expression and the survival time (Fig. 10A), the survival time became significantly shorter when FRβ (+) macrophage expression was observed (Fig. 10B). That is, metastasis and recurrence occur at high frequency in cases exhibiting high-level FRβ (+) macrophage expression, and the FRβ (+) macrophage expression is highly related with hematogenous metastasis (Example 5). This strongly suggests that the FRβ (+) macrophage expression plays a role in accelerating progression (i.e., invasion and metastasis) of pancreatic cancer.

### [Example 7]

### Growth inhibition of invasive pancreatic cancer by recombinant immunotoxin

Growth inhibition of the human pancreatic cancer cell line (Capan-1 M9) transplanted into nude mice by recombinant immunotoxins was examined. The designed experimental protocol is shown in Fig. 11. The recombinant immunotoxin (dsFv anti-FRβ-PE38) 10 µg was injected into the tumors of mice every day, and the effects of dsFv anti-FRβ-PE38 against the growth of pancreatic adenocarcinoma M9 were observed. The values are the mean ± SEM of tumor volumes in the groups. The risk rate, p, in comparison with the group to which VH-PE38 had been administered was 0.003.

As a result, tumor growth of the recombinant immunotoxin (dsFv anti-FRβ-PE38) group was significantly inhibited in comparison with control group (saline, VH-PE38) (p = 0.003) (Fig. 12). However, no differences were observed in terms of changes in mouse body weights among the 3 groups (Fig. 13). The fact that toxicity (side effects) of the recombinant immunotoxin was not observed indicates that the agent of the present invention selectively inhibits mouse FRβ (+) macrophage alone.

### [Example 8]

As the result that the detection of CD68-expressing macrophage and FRβ-expressing macrophage in a normal human pancreatic tissue was attempted, the expression of CD68-expressing macrophage was observed (Fig. 14A), but the expression of FRβ-expressing macrophage was not observed with a significant difference (Figs. 14B and 14C).

### [Example 9]

Distribution of the CD68 (+) macrophage and the FRβ (+) macrophage in the human pancreatic cancer tissue was studied by using fluorescence immunostaining. While the CD68 (+) macrophage was extensively distributed from the cancer front (borderline) toward the center of cancer tissue (Fig. 15A, CD68 (+) macrophages: indicated in red), the FRβ (+) macrophage was selectively distributed around the cancer front (Fig. 15A, FRβ (+) macrophages: indicated in green). In addition, the distribution of the CD68 (+) macrophage and the FRβ (+) macrophage in the human pancreatic cancer tissue was compared between the front and the center of pancreatic cancer. While the CD68 macrophage was substantially equally distributed in the front and the center of cancer, the distribution of the FRβ (+) macrophages was observed at a significant level selectively around the cancer front (Fig. 15B, Fig. 15C).

### [Example 10]

The high and low expressions of CD68 or FRβ in the CD68 (+) macrophage or FRβ (+) macrophage in the human pancreatic cancer tissues, the number of micro-blood vessels in tumor, and hematogenous metastasis in patients with pancreatic cancer, were compared and analyzed. As a result of the comparison between the group exhibiting a low CD68 expression in CD68 (+) macrophage and the group exhibiting a high CD68 expression, no significant differences were observed in terms of the number of micro-blood vessels in tumor and hematogenous metastasis. In the case of FRβ (+) macrophages, however, significant differences were observed in the number of micro-blood vessels in tumor and hematogenous metastasis (Fig. 16).

### [Example 11]

Expression of an angiogenesis factor (VEGF) in CD68 (+) macrophage and in FRβ (+) macrophage in the human pancreatic cancer tissue was analyzed. While the VEGF expression in FRβ (+) macrophage was high (Fig. 17A), the VEGF expression in CD68 macrophage was low (Fig. 17B). As a result of quantification, the VEGF expression was found to be significantly high in FRβ (+) macrophage (Fig. 17C). Specifically, FRβ (+) macrophage expresses the angiogenesis factor (VEGF) at a high level, whereby the FRβ (+) macrophage is thus highly related with a large number of micro-blodd vessels in tumor and with a high rate of hematogenous metastasis.

### [Example 12]

Quantities of each type of invasive macrophages were compared, and the survival curves of patients with pancreatic cancer depending on types of macrophages were examined. The number of macrophages becomes smaller from CD68 (+) macrophage, CD163 (+) macrophage, to FRβ (+) macrophage in that order (Fig. 18A). As a result of the comparison of the survival curves of patients with pancreatic cancer, no significant differences were observed in CD68 (+) macrophages (Fig. 18B, which is the same as Fig. 10A), significant differences were observed in the CD163 (+) macrophage (Fig. 18C), and the most significant differences were observed in the FRβ macrophage. A greater number of FRβ (+) macrophages observed in patients with pancreatic cancer indicates poorer prognosis (Fig. 18D, which is the same as Fig. 10B). The results demonstrate that the FRβ (+) macrophage expresses the angiogenesis factor and the like at high levels, and that the FRβ (+) macrophage is thus involved in progression (i.e., invasion and metastasis) of pancreatic cancer.

### Industrial Applicability

According to the present invention, the growth and/or metastasis of metastatic, highly malignant, invasive pancreatic cancer can be inhibited. Since such invasive pancreatic cancer can be detected, tailor-made therapy can also be realized.

According to the present invention, detection and treatment of progressive, invasive pancreatic cancer become possible. In addition, the molecular-targeted agent of the present invention is capable of inhibiting metastasis of pancreatic cancer exhibiting an extremely lower detection rate and a lower survival rate, compared with other cancers. Accordingly, the present invention is useful in the pharmaceutical industry.

All publications, patents, and patent applications cited herein are incorporated herein by reference in their entirety.

## Claims

1. A pharmaceutical composition for inhibiting the growth and/or metastasis of invasive pancreatic cancer in a subject, comprising a molecular-targeted anticancer agent and a pharmaceutically acceptable carrier, wherein the molecular-targeted anticancer agent is a conjugate of a toxin or cytotoxic agent and an antibody or fragment thereof which immunologically and specifically binds to a cell-surface folate receptor β (FRβ) protein of an FRβ (+) macrophage that exists around pancreatic cancer cells at the invasive front.

2. The pharmaceutical composition according to claim 1, wherein the antibody is a monoclonal antibody, a polyclonal antibody, a chimeric antibody, a single-stranded antibody, a multispecific antibody, or a fragment thereof.

3. The pharmaceutical composition according to claim 1 or 2, wherein the antibody is a human antibody or a humanized antibody.

4. The pharmaceutical composition according to any one of claims 1 to 3, wherein the antibody or a fragment thereof is at least one of (1) to (4) below:
(1) an antibody or fragment thereof, in which the amino acid sequences of CDR1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
(2) an antibody or fragment thereof, in which the amino acid sequences of CDR1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
(3) an antibody or fragment thereof, in which the amino acid sequences of CDR1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ; and
(4) an antibody or fragment thereof, in which the amino acid sequences of CDR1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively.

5. The pharmaceutical composition according to any one of claims 1 to 3, wherein the antibody or fragment thereof binds specifically to an epitope of a region selected from the group consisting of the amino acids 27-65, the amino acids 130-166, and the amino acids 206-233 of the amino acid sequence of SEQ ID NO: 1 of human FRβ protein.

6. The pharmaceutical composition according to any one of claims 1 to 5, wherein the toxin is a bacterium-derived toxin.

7. The pharmaceutical composition according to claim 6, wherein the bacterium-derived toxin is a Pseudomonas toxin, a diphteria toxin, or a staphylococcus toxin.

8. The pharmaceutical composition according to any one of claims 1 to 7, wherein the molecular-targeted anticancer agent is an immunotoxin.

9. The pharmaceutical composition according to any one of claims 1 to 5, wherein the cytotoxic agent is an antitumor agent, a tumor growth inhibitor, an apoptosis inducer for tumor cell, or a radioactive nuclide.

10. The pharmaceutical composition according to any one of claims 1 to 9, wherein the metastasis is lymph node metastasis or hematogenous metastasis.

11. The pharmaceutical composition according to any one of claims 1 to 10, wherein the subj ect is a human.

12. A method for examining the degree of malignancy of pancreatic cancer or the presence of invasive pancreatic cancer, comprising: bringing a pancreatic cancer tissue sample of a subject into contact with a labeled or non-labeled antibody or fragment thereof that specifically binds to the cell-surface FRβ protein of the FRβ (+) macrophage; measuring whether the FRβ (+) macrophage exists around the pancreatic cancer tissue at the invasive front based on the formation of a conjugate of the FRβ protein and the antibody or fragment thereof; and determining that the cancer tissue is invasive and metastatic when the FRβ (+) macrophage is distributed around pancreatic cancer cells at the invasive front.

13. The method according to claim 12, wherein the antibody is a monoclonal antibody or fragment thereof.

14. The method according to claim 12 or 13, wherein the antibody or fragment thereof is at least one of (1) to (5) below:
(1) an antibody or fragment thereof, in which the amino acid sequences of CDR1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
(2) an antibody or fragment thereof, in which the amino acid sequences of CDR1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
(3) an antibody or fragment thereof, in which the amino acid sequences of CDR1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ;
(4) an antibody or fragment thereof, in which the amino acid sequences of CDR1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively; and
(5) an antibody or fragment thereof binding specifically to an epitope of a region selected from the group consisting of the amino acids 27-65, the amino acids 130-166, and the amino acids 206-233 of the amino acid sequence of SEQ ID NO: 1 of human FRβ protein.

15. A diagnostic agent or kit for determining the degree of malignancy of pancreatic cancer or the presence of invasive pancreatic cancer, comprising an antibody or fragment thereof which immunologically and specifically binds to a cell-surface FRβ protein of the FRβ (+) macrophage that exists around pancreatic cancer cells at the invasive front.

16. A diagnostic agent or kit for image-diagnosing the presence of invasive pancreatic cancer in a subject, comprising a conjugate of a label and an antibody or fragment thereof that immunologically and specifically binds to a cell-surface FRβ protein of a FRβ (+) macrophage.

17. The diagnostic agent or kit according to claim 10, wherein the label is a fluorophore, pigment, or radioactive isotope.

18. The diagnostic agent or kit according to any one of claims 15 to 17, wherein the antibody or fragment thereof is at least one of (1) to (5) below:
(1) an antibody or fragment thereof, in which the amino acid sequences of CDR1, CDRL2, and CDRL3 of a light chain variable region (VL) are SEQ ID NOs: 8, 9, and 10 respectively, and the amino acid sequences of CDRH1, CDRH2, and CDRH3 of a heavy chain variable region (VH) are SEQ ID NOs: 11, 12, and 13 respectively;
(2) an antibody or fragment thereof, in which the amino acid sequences of CDR1, CDRL2, and CDRL3 of a light chain variable region (VL) are SEQ ID NOs: 18, 19, and 20 respectively, and the amino acid sequences of CDRH1, CDRH2, and CDRH3 of a heavy chain variable region (VH) are SEQ ID NOs: 21, 22, and 23 repectively;
(3) an antibody or fragment thereof, in which the amino acid sequences of CDR1, CDRL2, and CDRL3 of a light chain variable region (VL) are SEQ ID NOs: 28, 29, and 30 respectively, and the amino acid sequences of CDRH1, CDRH2, and CDRH3 of a heavy chain variable region (VH) are SEQ ID NOs: 31, 32, and 33 repectively ;
(4) an antibody or fragment thereof, in which the amino acid sequences of CDR1, CDRL2, and CDRL3 of a light chain variable region (VL) are SEQ ID NOs: 38, 39, and 40 repectively, and the amino acid sequences of CDRH1, CDRH2, and CDRH3 of a heavy chain variable region (VH) are SEQ ID NOs: 41, 42, and 43 repectively; and
(5) an antibody or fragment thereof binding specifically to an epitope of a region selected from the group consisting of the amino acids 27-65, the amino acids 130-166, and the amino acids 206-233 of the amino acid sequence of SEQ ID NO: 1 of human FRβ protein.


SEQUENCE LISTING

<110>  Kagoshima University

<120>  Comositions for treatment and diagnosis of pancreatic cancer

<130>  PH-5151-PCT

<150>  JP 2011-061362
<151>  2011-03-18

<160>  63

<170>  PatentIn version 3.5

<210>  1
<211>  233
<212>  PRT
<213>  Homo sapiens

<400>  1

Asp Arg Thr Asp Leu Leu Asn Val Cys Met Asp Ala Lys His His Lys
1               5                   10                  15

Thr Lys Pro Gly Pro Glu Asp Lys Leu His Asp Gln Cys Ser Pro Trp
            20                  25                  30

Lys Lys Asn Ala Cys Cys Thr Ala Ser Thr Ser Gln Glu Leu His Lys
        35                  40                  45

Asp Thr Ser Arg Leu Tyr Asn Phe Asn Trp Asp His Cys Gly Lys Met
    50                  55                  60

Glu Pro Ala Cys Lys Arg His Phe Ile Gln Asp Thr Cys Leu Tyr Glu
65                  70                  75                  80

Cys Ser Pro Asn Leu Gly Pro Trp Ile Gln Gln Val Asn Gln Thr Trp
                85                  90                  95

Arg Lys Glu Arg Phe Leu Asp Val Pro Leu Cys Lys Glu Asp Cys Gln
            100                 105                 110

Arg Trp Trp Glu Asp Cys His Thr Ser His Thr Cys Lys Ser Asn Trp
        115                 120                 125

His Arg Gly Trp Asp Trp Thr Ser Gly Val Asn Lys Cys Pro Ala Gly
    130                 135                 140

Ala Leu Cys Arg Thr Phe Glu Ser Tyr Phe Pro Thr Pro Ala Ala Leu
145                 150                 155                 160

Cys Glu Gly Leu Trp Ser His Ser Tyr Lys Val Ser Asn Tyr Ser Arg
                165                 170                 175

Gly Ser Gly Arg Cys Ile Gln Met Trp Phe Asp Ser Ala Gln Gly Asn
            180                 185                 190

Pro Asn Glu Glu Val Ala Arg Phe Tyr Ala Ala Ala Met His Val Asn
        195                 200                 205

Ala Gly Glu Met Leu His Gly Thr Gly Gly Leu Leu Leu Ser Leu Ala
    210                 215                 220

Leu Met Leu Gln Leu Trp Leu Leu Gly
225                 230

<210> 2
<211> 1140
<212> DNA

<213>  Homo sapiens

<400>  2
   gggagagcgc gttgtctacc ctgtaccgaa gacagaggct gtggggacag cctaggggcc      60
   tggatctatt gcctacttag agagaggcca actcagacac agccgtgtat gctcccagca     120
   gcaacggagg ttcagctccg cctgcaggga cagaaagaca tggtctggaa atggatgcca     180
   cttctgctgc ttctggtctg tgtagccacc atgtgcagtg cccaggacag gactgatctc     240
   ctcaatgtct gtatggatgc caagcaccac aagacaaagc caggtcctga ggacaagctg     300
   catgaccaat gcagtccctg gaagaagaat gcctgctgca cagccagcac cagccaggag     360
   ctgcacaagg acacctcccg cctgtacaac tttaactggg accactgcgg caagatggag     420
   cccgcctgca agcgccactt catccaggac acctgtctct atgagtgctc acccaacctg     480
   gggccctgga tccagcaggt gaatcagagc tggcgcaaag aacgcttcct ggatgtgccc     540
   ttatgcaaag aggactgtca gcgctggtgg gaggattgtc acacctccca cacgtgcaag     600
   agcaactggc acagaggatg ggactggacc tcaggagtta acaagtgccc agctggggct     660
   ctctgccgca cctttgagtc ctacttcccc actccagctg ccctttgtga aggcctctgg     720
   agtcactcat acaaggtcag caactacagc cgagggagcg gccgctgcat ccagatgtgg     780
   tttgattcag cccagggcaa ccccaacgag gaagtggcga ggttctatgc tgcagccatg     840
   catgtgaatg ctggtgagat gcttcatggg actgggggtc tcctgctcag tctggccctg     900
   atgctgcaac tctggctcct tggctgagtt cagtcctccc agactacctg ccctcagctt     960
   ggataaccag gctgggctca gctcagctcc cacaaatgac agccccttaa gcatgcttct    1020
   attagtcacc taaccctctg tcacccagtc tgttgctgct ccatggtggg gccaagagtc    1080
   acttctaata aacagactgt tttctaaaaa aaaaaaaaaa aaaaaaaaaa aaaaacatgt    1140

<210> 3
<211> 229
<212> PRT
<213> Homo sapiens

<400> 3

Ala Arg Thr Glu Leu Leu Asn Val Cys Met Asn Ala Lys His His Lys
1               5                   10                  15

Glu Lys Pro Gly Pro Glu Asp Lys Leu His Glu Gln Cys Arg Pro Trp
            20                  25                  30

Arg Lys Asn Ala Cys Cys Ser Thr Asn Thr Ser Gln Glu Ala His Lys
        35                  40                  45

Asp Val Ser Tyr Leu Tyr Arg Phe Asn Trp Asn His Cys Gly Glu Met
    50                  55                  60

Ala Pro Ala Cys Lys Arg His Phe Ile Gln Asp Thr Cys Leu Tyr Glu
65                  70                  75                  80

Cys Ser Pro Asn Leu Gly Pro Trp Ile Gln Gln Val Asp Gln Ser Trp
                85                  90                  95

Arg Lys Glu Arg Val Leu Asn Val Pro Leu Cys Lys Glu Asp Cys Glu
            100                 105                 110

Gln Trp Trp Glu Asp Cys Arg Thr Ser Tyr Thr Cys Lys Ser Asn Trp
        115                 120                 125

His Lys Gly Trp Asn Trp Thr Ser Gly Phe Asn Lys Cys Ala Val Gly
    130                 135                 140

Ala Ala Cys Gln Pro Phe His Phe Tyr Phe Pro Thr Pro Thr Val Leu
145                 150                 155                 160

Cys Asn Glu Ile Trp Thr His Ser Tyr Lys Val Ser Asn Tyr Ser Arg
                165                 170                 175

Gly Ser Gly Arg Cys Ile Gln Met Trp Phe Asp Pro Ala Gln Gly Asn
            180                 185                 190

Pro Asn Glu Glu Val Ala Arg Phe Tyr Ala Ala Ala Met Ser Gly Ala
        195                 200                 205

Phe Leu Gly Pro Trp Ala Ala Trp Pro Leu Ser Leu Ala Leu Met Leu
    210                 215                 220

Leu Trp Leu Leu Ser
225

<210>  4
<211>  109
<212>  PRT
<213>  Mus musculus

<400>  4

Asp Ile Gln Met Thr Gln Ser Ser Ser Ser Phe Ser Val Ser Leu Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Asn Ala Pro Arg Leu Leu Ile
        35                  40                  45

Ser Gly Ala Thr Ser Leu Glu Thr Gly Val Pro Leu Arg Phe Ser Gly
    50                  55                  60

Ser Gly Ser Gly Lys Asp Tyr Thr Leu Ser Ile Thr Ser Leu Gln Thr
65                  70                  75                  80

Glu Asp Ile Ala Thr Tyr Tyr Cys Gln Gln Tyr Trp Ser Thr Pro Trp
                85                  90                  95

Thr Phe Gly Gly Gly Thr Lys Leu Glu Ile Lys Lys Ala
            100                 105

<210>  5
<211>  328
<212>  DNA
<213>  Mus musculus

<400>  5
gacatccaga tgacacaatc ttcatcctcc ttttctgtat ctctaggaga cagagtcacc      60

attacttgca aggcaagtga ggacatatat aatcggttag cctggtatca gcagaaacca     120

ggaaatgctc ctaggctctt aatatctggt gcaaccagtt tggaaactgg ggttccttta     180

agattcagtg gcagtggatc tggaaaggat tacactctca gcattaccag tcttcagact     240

gaagatattg ctacttatta ctgtcaacag tattggagta ctccgtggac gttcggtgga     300

ggcaccaagc tggaaatcaa aaaagctt                                        328

<210>  6
<211>  121
<212>  PRT
<213>  Mus musculus

<400> 6

Gln Val Gln Leu Gln Gln Ser Gly Ala Glu Leu Met Lys Pro Gly Ala
1               5                   10                  15

Ser Leu Lys Ile Ser Cys Lys Ala Thr Gly Tyr Thr Phe Ser Ser Tyr
            20                  25                  30

Trp Ile Glu Trp Val Lys Gln Arg Pro Gly His Gly Leu Gln Trp Ile
        35                  40                  45

Gly Glu Ile Leu Pro Gly Ser Gly Ser Ser Asn Tyr Asn Glu Lys Phe
    50                  55                  60

Lys Gly Lys Ala Thr Phe Thr Ala Asp Thr Ser Ser Asn Thr Ala Tyr
65                  70                  75                  80

Met Gln Leu Ser Ser Leu Thr Ser Glu Asp Ser Ala Val Tyr Tyr Cys
                85                  90                  95

Ala Arg Arg Gly Ser Ser Gly Ser Tyr Phe Ala Met Asp Tyr Trp Gly
            100                 105                 110

Gln Gly Thr Ser Val Thr Val Ser Ser
        115                 120

<210> 7
<211> 375
<212> DNA
<213> Mus musculus

<400> 7
caggttcagc tgcagcagtc tggagctgag ctgatgaagc ctggggcctc actgaagata        60

tcctgcaagg ctactggcta cacattcagt agctactgga tagagtgggt aaagcagagg 120

cctggacatg gccttcagtg gattggagaa attttgcctg gaagtggtag ttctaactac 180

aatgagaagt tcaagggcaa ggccacattc actgcagata catcctccaa cacagcctac 240

atgcaactca gcagcctgac atctgaggac tctgccgtct attactgtgc aagacggggc 300

agctcgggct cttactttgc tatggactac tggggtcaag gaacctcagt caccgtctcc 360

tcataggaat tcggc 375

<210> 8
<211> 11
<212> PRT
<213> Mus musculus

<400> 8

Lys Ala Ser Glu Asp Ile Tyr Asn Arg Leu Ala
1               5                   10

<210> 9
<211> 7
<212> PRT
<213> Mus musculus

<400> 9

Gly Ala Thr Ser Leu Glu Thr
1               5

<210> 10
<211> 9
<212> PRT
<213> Mus musculus

<400> 10

Gln Gln Tyr Trp Ser Thr Pro Trp Thr
1               5

<210>  11
<211>  5
<212>  PRT
<213>  Mus musculus

<400>  11

Ser Tyr Trp Ile Glu
1               5

<210>  12
<211>  17
<212>  PRT
<213>  Mus musculus

<400>  12

Glu Ile Leu Pro Gly Ser Gly Ser Ser Asn Tyr Asn Glu Lys Phe Lys
1               5                   10                  15

Gly

<210>  13
<211>  12
<212>  PRT
<213>  Mus musculus

<400>  13

Arg Gly Ser Ser Gly Ser Tyr Phe Ala Met Asp Tyr
1               5                   10

<210>  14

<211>  150
<212>  PRT
<213>  Mus musculus

<400>  14

Met Val Leu Ile Leu Leu Leu Leu Trp Val Ser Gly Glu Lys Phe Lys
1               5                   10                  15

Gly Thr Cys Gly Asp Ile Val Met Ser Gln Ser Pro Ser Ser Leu Ala
            20                  25                  30

Val Ser Ala Gly Glu Lys Val Thr Met Ser Cys Lys Ser Ser Gln Ser
        35                  40                  45

Leu Leu Asn Ser Arg Thr Arg Lys Thr Tyr Leu Ala Trp Tyr Gln Gln
    50                  55                  60

Lys Pro Gly Gln Ser Pro Lys Leu Leu Ile Tyr Trp Ala Ser Thr Arg
65                  70                  75                  80

Glu Ser Gly Val Pro Asp Arg Phe Thr Gly Ser Gly Ser Gly Thr Glu
                85                  90                  95

Phe Thr Leu Thr Ile Ser Ser Val Gln Ala Glu Asp Leu Ala Val Tyr
            100                 105                 110

Tyr Cys Lys Gln Ser Tyr Asn Leu Trp Thr Phe Gly Gly Gly Thr Lys
        115                 120                 125

Leu Glu Ile Lys Arg Ala Asp Ala Ala Pro Thr Val Ser Ile Phe Pro
    130                 135                 140

Pro Ser Ser Lys Leu Gly
145                 150

<210>  15
<211>  450
<212>  DNA
<213>  Mus musculus

<400>  15
atggttctca tcttgctgct gctatgggta tctggtgaga aatttaaagg tacctgtggg      60

gacattgtga tgtcacaatc tccatcctcc ctggctgtgt cagcaggaga gaaggtcact     120

atgagctgca aatccagtca gagtctgctc aacagtagaa cccgaaagac ctacttggct     180

tggtatcagc agaaaccagg gcagtctcct aaactgctga tctactgggc atccactagg     240

gaatctgggg tccctgatcg cttcacaggc agtggatctg ggacagagtt cactctcacc     300

atcagcagtg tgcaggctga agacctggca gtttattact gcaagcaatc ttataatctg     360

tggacgttcg gtggaggcac caagctggaa atcaaacggg ctgatgctgc accaactgta     420

tccatcttcc cgccatccag taagctggga                                      450

<210>  16
<211>  149
<212>  PRT
<213>  Mus musculus

<400>  16

Met Glu Trp Thr Trp Val Ile Leu Phe Leu Met Ala Val Val Thr Gly
1               5                   10                  15

Val Asn Ser Glu Val Gln Leu Gln Gln Ser Gly Ala Glu Leu Val Lys
            20                  25                  30

Pro Gly Ala Ser Val Arg Leu Ser Cys Thr Ala Ser Gly Phe Asn Ile
        35                  40                  45

Lys Asp Thr Tyr Met Tyr Trp Val Lys Gln Arg Pro Glu Gln Gly Leu
    50                  55                  60

Glu Trp Ile Gly Arg Leu Asp Pro Ala Asn Gly Asn Thr Arg Tyr Asp
65                  70                  75                  80

Pro Lys Phe Gln Gly Lys Ala Thr Ile Thr Ser Asp Thr Ser Ser Asn
                85                  90                  95

Thr Ala Tyr Leu Gln Leu Ser Ser Leu Thr Ser Glu Asp Thr Ala Val
            100                 105                 110

Tyr Tyr Cys Ala Gly Asp Tyr Pro Ile Ile Asp Tyr Trp Gly Gln Gly
        115                 120                 125

Thr Thr Leu Thr Val Ser Ser Ala Lys Thr Thr Pro Pro Pro Val Tyr
    130                 135                 140

Pro Leu Ala Pro Gly
145

<210>  17
<211>  447
<212>  DNA
<213>  Mus musculus

<400>  17
atggaatgga cctgggtcat tctcttcctg atggcagtgg ttacaggggt caattcagag        60

gttcagctgc agcagtctgg ggcagaactt gtgaagccag gggcctcagt caggttgtcc       120

tgcacagctt ctggcttcaa cattaaagac acctacatgt actgggtgaa acagaggcct 180

gaacagggcc tggagtggat tggaaggctt gatcctgcga atggtaatac tagatatgac 240

ccgaagttcc agggcaaggc cactataaca tcagacacat cctccaacac agcctacctg 300

caactcagca gcctgacatc tgaggacact gccgtctatt actgtgcggg ggactacccg 360

ataattgact actggggcca aggcaccact ctcacagtct cctcagccaa aacgacaccc 420

ccacccgtct atccactggc ccctgga 447

<210> 18
<211> 17
<212> PRT
<213> Mus musculus

<400> 18

Lys Ser Ser Gln Ser Leu Leu Asn Ser Arg Thr Arg Lys Thr Tyr Leu
1               5                   10                  15

Ala

<210> 19
<211> 7
<212> PRT
<213> Mus musculus

<400> 19

Trp Ala Ser Thr Arg Glu Ser
1               5

<210> 20
<211> 8
<212> PRT

<213>  Mus musculus

<400>  20

Lys Gln Ser Tyr Asn Leu Trp Thr
1               5

<210>  21
<211>  5
<212>  PRT
<213>  Mus musculus

<400>  21

Asp Thr Tyr Met Tyr
1               5

<210>  22
<211>  17
<212>  PRT
<213>  Mus musculus

<400>  22

Arg Leu Asp Pro Ala Asn Gly Asn Thr Arg Tyr Asp Pro Lys Phe Gln
1               5                   10                  15

Gly

<210>  23
<211>  7
<212>  PRT
<213>  Mus musculus

<400>  23

Asp Tyr Gly Ile Ile Asp Tyr

1               5


<210>  24
<211>  116
<212>  PRT
<213>  Rattus norvegicus

<400>  24

His Met Asp Ile Val Thr Gln Ser Pro Ser Ser Leu Ala Val Ser Ala
1               5                   10                  15

Gly Glu Thr Val Thr Ile Asn Cys Lys Ser Ser Gln Ser Leu Leu Tyr
            20                  25                  30

Ser Gly Asn Gln Lys Asn Tyr Leu Ala Trp Tyr Gln Gln Lys Pro Gly
        35                  40                  45

Gln Ser Pro Lys Leu Leu Ile Tyr Trp Ala Ser Thr Arg Gln Ser Gly
    50                  55                  60

Val Pro Asp Arg Phe Ile Gly Ser Gly Ser Gly Thr Asp Phe Thr Leu
65                  70                  75                  80

Thr Ile Ser Ser Val Gln Ala Glu Asp Leu Ala Ile Tyr Tyr Cys Gln
                85                  90                  95

Gln Tyr Tyr Asp Thr Pro Leu Thr Phe Gly Cys Gly Thr Lys Leu Glu
            100                 105                 110

Ile Lys Glu Phe
        115

<210>  25
<211>  351
<212>  DNA
<213>  Rattus norvegicus

<400>  25
catatggaca ttgtgaccca atctccatcc tctctggctg tgtcagcagg agagacggtc      60

actataaact gcaagtccag tcagagtctt ttatacagtg gaaaccaaaa gaactacttg      120

gcctggtacc agcagaaacc agggcagtct cctaaactgc tgatctactg ggcatctact      180

aggcaatctg gtgtccctga tcgcttcata ggcagtggat ctgggacaga cttcactctg      240

accatcagca gtgtgcaggc agaagatctg gcaatttatt actgtcagca gtattatgat      300

actccgctca cgttcggttg tggcaccaag ctggaaatca aataggaatt c      351

<210>  26
<211>  163
<212>  PRT
<213>  Rattus norvegicus

<400>  26

Met Lys Phe Gly Phe Ser Leu Val Phe Leu Val Leu Phe Ile Lys Gly
1               5                   10                  15

Val Gln Cys Glu Val Gln Leu Val Glu Ser Gly Gly Gly Leu Val Gln
            20                  25                  30

Pro Gly Arg Ser Leu Lys Leu Ser Cys Ala Ala Ser Gly Phe Thr Phe
        35                  40                  45

Ser Asn Tyr Asp Met Ala Trp Val Arg Gln Ala Pro Asp Thr Lys Cys
    50                  55                  60

Leu Glu Trp Val Ala Ser Ile Ser Pro Ser Gly Asp Ile Thr Tyr Tyr
65                  70                  75                  80

Arg Asp Ser Val Lys Gly Arg Phe Thr Val Ser Arg Asp Asn Thr Lys
                85                  90                  95

Ser Thr Leu Phe Leu Gln Met Asp Ser Leu Arg Ser Glu Asp Thr Ala
            100                 105                 110

Thr Tyr Tyr Cys Ala Arg His Gly Pro Thr Glu Ala Pro Asp Ile Met
        115                 120                 125

Asp Ala Trp Gly Gln Gly Ala Ser Val Thr Val Ser Ser Ala Glu Thr
    130                 135                 140

Thr Ala Pro Ser Val Tyr Pro Leu Ala Pro Gly Thr Ala Leu Lys Ser
145                 150                 155                 160

Asn Met Val

<210>  27
<211>  490
<212>  DNA
<213>  Rattus norvegicus

<400>  27
atgaagttcg ggttcagctt ggttttcctt gtccttttca taaaaggtgt ccagtgtgag      60

gtgcagctgg tggagtctgg gggaggctta gtgcagcctg gaaggtccct gaaactctcc      120

tgtgcagcct caggattcac tttcagtaac tatgacatgg cctgggtccg ccaggctcca      180

gatacgaagt gtctggagtg ggtcgcatcc attagtccta gtggtgatat cacttactat      240

cgagactccg tgaagggccg attcactgtc tccagagata atacaaaaag caccctattc 300

ctgcaaatgg acagtctgag gtctgaggac acggccactt attactgtgc aagacatggg 360

cctacggagg cccccgatat tatggatgcc tggggtcaag gagcttcagt cactgtctcc 420

tcagctgaaa caacagcccc atctgtctat ccactggctc ctggaactgc tctcaaaagt 480

aacatggtga 490

<210> 28
<211> 17
<212> PRT
<213> Rattus norvegicus

<400> 28

Lys Ser Ser Gln Ser Leu Leu Tyr Ser Gly Asn Gln Lys Asn Tyr Leu
1               5                   10                  15

Ala

<210> 29
<211> 7
<212> PRT
<213> Rattus norvegicus

<400> 29

Trp Ala Ser Thr Arg Gln Ser
1               5

<210> 30
<211> 9
<212> PRT
<213> Rattus norvegicus

<400>  30

Gln Gln Tyr Tyr Asp Thr Pro Leu Thr
1               5

<210>  31
<211>  5
<212>  PRT
<213>  Rattus norvegicus

<400>  31

Asn Tyr Asp Met Ala
1               5

<210>  32
<211>  17
<212>  PRT
<213>  Rattus norvegicus

<400>  32

Ser Ile Ser Pro Ser Gly Asp Ile Thr Tyr Tyr Arg Asp Ser Val Lys
1               5                   10                  15

Gly

<210>  33
<211>  12
<212>  PRT
<213>  Rattus norvegicus

<400>  33

His Gly Pro Thr Glu Ala Pro Asp Ile Met Asp Ala
1               5                   10

<210> 34
<211> 208
<212> PRT
<213> Rattus norvegicus

<400> 34

Asp Ile Val Met Thr Gln Ser Pro Ser Ser Leu Ala Val Ser Ala Gly
1               5                   10                  15

Glu Thr Val Thr Ile Asn Cys Lys Ser Ser Gln Ser Leu Leu Tyr Ser
            20                  25                  30

Gly Asn Gln Lys Asn Tyr Leu Ala Trp Tyr Gln Gln Lys Pro Gly Gln
        35                  40                  45

Ser Pro Lys Leu Leu Ile His Trp Ala Ser Thr Arg Gln Ser Gly Val
    50                  55                  60

Pro Asp Arg Phe Ile Gly Ser Gly Ser Gly Thr Asp Phe Thr Leu Thr
65                  70                  75                  80

Ile Ser Ser Val Gln Ala Glu Asp Leu Ala Ile Tyr Tyr Cys Gln Gln
                85                  90                  95

Tyr Tyr Asp Ser Pro Leu Thr Phe Gly Ser Gly Thr Lys Leu Glu Ile
            100                 105                 110

Lys Arg Ala Asp Ala Ala Pro Thr Val Ser Ile Phe Pro Pro Ser Thr
        115                 120                 125

Glu Gln Leu Ala Thr Gly Gly Ala Ser Val Val Cys Leu Met Asn Asn
    130                 135                 140

Phe Tyr Pro Arg Asp Ile Ser Val Lys Trp Lys Ile Asp Gly Thr Glu
145                 150                 155                 160

Arg Arg Asp Gly Val Leu Asp Ser Val Thr Asp Gln Asp Ser Lys Asp
                165                 170                 175

Ser Thr Tyr Ser Met Ser Ser Thr Leu Ser Leu Thr Lys Ala Asp Tyr
            180                 185                 190

Glu Ser His Asn Leu Tyr Thr Cys Glu Val Val His Lys Thr Ser Ser
        195                 200                 205

<210> 35
<211> 627
<212> DNA
<213> Rattus norvegicus

<400> 35
gacattgtga tgacccaatc tccatcctct ctggctgtgt cagcaggaga gacggtcact      60

ataaactgca agtccagtca gagtctttta tacagtggaa accaaaagaa ctacttggcc     120

tggtaccagc agaaaccagg gcagtctcct aaactgctga tccactgggc atctactagg     180

caatctggtg tccctgatcg cttcataggc agtggatctg ggacagactt cactctgacc     240

atcagcagtg tgcaggcaga agatctggca atttattact gtcagcagta ttatgatagt     300

ccgctcacgt tcggttctgg gaccaagctg gagatcaaac gggctgatgc tgcaccaact     360

gtatctatct tcccaccatc cacggaacag ttagcaactg gaggtgcctc agtcgtgtgc     420

ctcatgaaca acttctatcc cagagacatc agtgtcaagt ggaagattga tggcactgaa     480

cgacgagatg gtgtcctgga cagtgttact gatcaggaca gcaaagacag cacgtacagc     540

atgagcagca ccctctcgtt gaccaaggct gactatgaaa gtcataacct ctatacctgt 600

gaggttgttc ataagacatc atcctaa 627

<210> 36
<211> 179
<212> PRT
<213> Rattus norvegicus

<400> 36

Met Lys Phe Gly Phe Ser Leu Val Phe Leu Val Leu Phe Ile Lys Gly
1               5                   10                  15

Val Gln Cys Glu Val Gln Leu Val Glu Ser Gly Gly Gly Leu Val Gln
            20                  25                  30

Pro Gly Arg Ser Leu Lys Leu Ser Cys Ala Ala Ser Gly Phe Thr Phe
        35                  40                  45

Ser Asn Tyr Asp Met Ala Trp Val Arg Gln Ala Pro Thr Lys Gly Leu
    50                  55                  60

Glu Trp Val Ala Ser Ile Ser Pro Ser Gly Gly Ile Pro Tyr Tyr Arg
65                  70                  75                  80

Asp Ser Val Lys Gly Arg Phe Thr Val Ser Arg Asp Asn Ala Lys Ser
                85                  90                  95

Thr Leu Tyr Leu Gln Met Asp Ser Leu Arg Ser Glu Asp Thr Ala Thr
            100                 105                 110

Tyr Tyr Cys Ala Arg His Gly Pro Thr Glu Ala Pro Asp Val Met Asp
        115                 120                 125

Ala Trp Gly Gln Gly Ala Ser Val Thr Val Ser Ser Ala Glu Thr Thr
    130                 135                 140

Ala Pro Ser Val Tyr Pro Leu Ala Pro Gly Thr Ala Leu Lys Ser Asn
145                 150                 155                 160

Ser Met Val Lys Trp Asp Pro Ser Arg Ser Thr Gly Asn Ser Asn Trp
                165                 170                 175

Arg Thr Gly

<210>  37
<211>  539
<212>  DNA
<213>  Rattus norvegicus

<400>  37
atgaagttcg ggttcagctt ggttttcctt gtccttttca taaaaggtgt ccagtgtgag      60

gtgcagctgg tggagtctgg gggaggctta gtgcagcctg gaaggtccct gaaactctcc     120

tgtgcagcct caggattcac tttcagtaac tatgacatgg cctgggtccg ccaggctcca     180

acgaagggtc tggagtgggt cgcatccatt agtcctagtg gtggtatccc ttactatcga     240

gactccgtga agggccgatt cactgtctcc agagataatg caaaaagcac cctatacctg     300

caaatggaca gtctgaggtc tgaggacacg gccacttatt actgtgcaag acatgggcct     360

acggaggccc ccgatgttat ggatgcctgg ggtcaaggag cttcagtcac tgtctcctca     420

gctgaaacaa cagccccatc tgtctatcca ctggctcctg gaactgctct caaaagtaac     480

tccatggtga aatgggatcc gtctagatcg acgggcaaca gcaactggcg aacaggcaa      539

<210>  38
<211>  17
<212>  PRT
<213>  Rattus norvegicus

<400>  38

Lys Ser Ser Gln Ser Leu Leu Tyr Ser Gly Asn Gln Lys Asn Tyr Leu
1               5                   10                  15

Ala

<210>  39
<211>  7
<212>  PRT
<213>  Rattus norvegicus

<400>  39

Trp Ala Ser Thr Arg Gln Ser
1               5

<210>  40
<211>  9
<212>  PRT
<213>  Rattus norvegicus

<400>  40

Gln Gln Tyr Tyr Asp Ser Pro Leu Thr
1               5

<210>  41
<211>  5
<212>  PRT
<213>  Rattus norvegicus

<400>  41

Asn Tyr Asp Met Ala
1               5

<210>  42
<211>  17
<212>  PRT
<213>  Rattus norvegicus

<400>  42

Ser Ile Ser Pro Ser Gly Gly Ile Pro Tyr Tyr Arg Asp Ser Val Lys
1               5                   10                  15

Gly

<210>  43
<211>  12
<212>  PRT
<213>  Rattus norvegicus

<400>  43

His Gly Pro Thr Glu Ala Pro Asp Val Met Asp Ala
1               5                   10

<210>  44
<211>  468
<212>  PRT
<213>  Artificial

<220>
<223>  synthetic

<400>  44

Glu Val Gln Leu Gln Gln Ser Gly Ala Glu Leu Val Lys Pro Gly Ala
1               5                   10                  15

Ser Val Arg Leu Ser Cys Thr Ala Ser Gly Phe Asn Ile Lys Asp Thr
            20                  25                  30

Tyr Met Tyr Trp Val Lys Gln Arg Pro Glu Gln Cys Leu Glu Trp Ile
        35                  40                  45

Gly Arg Leu Asp Pro Ala Asn Gly Asn Thr Arg Tyr Asp Pro Lys Phe
    50                  55                  60

Gln Gly Lys Ala Thr Ile Thr Ser Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Gly Asp Tyr Gly Ile Ile Asp Tyr Trp Gly Gln Gly Thr Thr Leu
            100                 105                 110

Thr Val Ser Ser Lys Ala Ser Gly Gly Pro Glu Gly Gly Ser Leu Ala
        115                 120                 125

Ala Leu Thr Ala His Gln Ala Cys His Leu Pro Leu Glu Thr Phe Thr
    130                 135                 140

Arg His Arg Gln Pro Arg Gly Trp Glu Gln Leu Glu Gln Cys Gly Tyr
145                 150                 155                 160

Pro Val Gln Arg Leu Val Ala Leu Tyr Leu Ala Ala Arg Leu Ser Trp

                165                 170                 175

Asn Gln Val Asp Gln Val Ile Arg Asn Ala Leu Ala Ser Pro Gly Ser
            180                 185                 190

Gly Gly Asp Leu Gly Glu Ala Ile Arg Glu Gln Pro Glu Gln Ala Arg
        195                 200                 205

Leu Ala Leu Thr Leu Ala Ala Ala Glu Ser Glu Arg Phe Val Arg Gln
    210                 215                 220

Gly Thr Gly Asn Asp Glu Ala Gly Ala Ala Asn Gly Pro Ala Asp Ser
225                 230                 235                 240

Gly Asp Ala Leu Leu Glu Arg Asn Val Pro Thr Gly Ala Glu Phe Leu
                245                 250                 255

Gly Asp Gly Gly Asp Val Ser Phe Ser Thr Arg Gly Thr Gln Asn Trp
            260                 265                 270

Thr Val Glu Arg Leu Leu Gln Ala His Arg Gln Leu Glu Glu Arg Val
        275                 280                 285

Tyr Val Phe Val Gly Tyr His Gly Thr Phe Leu Glu Ala Ala Gln Ser
    290                 295                 300

Ile Val Phe Gly Gly Val Arg Ala Arg Ser Gln Asp Leu Asp Ala Ile
305                 310                 315                 320

Trp Arg Gly Phe Tyr Ile Ala Gly Asp Pro Ala Leu Ala Tyr Gly Tyr
                325                 330                 335

Ala Gln Asp Gln Glu Pro Asp Ala Arg Gly Arg Ile Arg Asn Gly Ala
            340                 345                 350

Leu Leu Arg Val Tyr Val Pro Arg Ser Ser Leu Pro Gly Phe Tyr Arg
        355                 360                 365

Thr Ser Leu Thr Leu Ala Ala Pro Glu Ala Ala Gly Glu Val Glu Arg
    370                 375                 380

Leu Ile Gly His Pro Leu Pro Leu Arg Leu Asp Ala Ile Thr Gly Pro
385                 390                 395                 400

Glu Glu Glu Gly Gly Arg Leu Glu Thr Ile Leu Gly Trp Pro Leu Ala
                405                 410                 415

Glu Arg Thr Val Val Ile Pro Ser Ala Ile Pro Thr Asp Pro Arg Asn
            420                 425                 430

Val Gly Gly Asp Leu Asp Pro Ser Ser Ile Pro Asp Lys Glu Gln Ala
        435                 440                 445

Ile Ser Ala Leu Pro Asp Tyr Ala Ser Gln Pro Gly Lys Pro Pro Arg
    450                 455                 460

Glu Asp Leu Lys
465

<210>  45
<211>  112
<212>  PRT
<213>  Artificial

<220>
<223>  synthetic

<400>  45

Asp Ile Val Met Ser Gln Ser Pro Ser Ser Leu Ala Val Ser Ala Gly
1               5                   10                  15

Glu Lys Val Thr Met Ser Cys Lys Ser Ser Gln Ser Leu Leu Asn Ser
            20                  25                  30

Arg Thr Arg Lys Thr Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Glu Phe Thr Leu Thr
65                  70                  75                  80

Ile Ser Ser Val Gln Ala Glu Asp Leu Ala Val Tyr Tyr Cys Lys Gln
                85                  90                  95

Ser Tyr Asn Leu Trp Thr Phe Gly Cys Gly Thr Lys Leu Glu Ile Lys
            100                 105                 110

<210>  46
<211>  473
<212>  PRT
<213>  Artificial

<220>
<223>  synthetic

<400>  46

Gln Val Gln Leu Gln Gln Ser Gly Ala Glu Leu Met Lys Pro Gly Ala
1               5                   10                  15

Ser Leu Lys Ile Ser Cys Lys Ala Thr Gly Tyr Thr Phe Ser Ser Tyr
            20                  25                  30

Trp Ile Glu Trp Val Lys Gln Arg Pro Gly His Gly Leu Gln Trp Ile
        35                  40                  45

Gly Glu Ile Leu Pro Gly Ser Gly Ser Ser Asn Tyr Asn Glu Lys Phe
    50                  55                  60

Lys Gly Lys Ala Thr Phe Thr Ala Asp Thr Ser Ser Asn Thr Ala Tyr
65                  70                  75                  80

Met Gln Leu Ser Ser Leu Thr Ser Glu Asp Ser Ala Val Tyr Tyr Cys
                85                  90                  95

Ala Arg Arg Gly Ser Ser Gly Ser Tyr Phe Ala Met Asp Tyr Trp Gly
            100                 105                 110

Gln Gly Thr Ser Val Thr Val Ser Ser Lys Ala Ser Gly Gly Pro Glu
        115                 120                 125

Gly Gly Ser Leu Ala Ala Leu Thr Ala His Gln Ala Cys His Leu Pro
    130                 135                 140

Leu Glu Thr Phe Thr Arg His Arg Gln Pro Arg Gly Trp Glu Gln Leu
145                 150                 155                 160

Glu Gln Cys Gly Tyr Pro Val Gln Arg Leu Val Ala Leu Tyr Leu Ala

                165                 170                 175

Ala Arg Leu Ser Trp Asn Gln Val Asp Gln Val Ile Arg Asn Ala Leu
            180                 185                 190

Ala Ser Pro Gly Ser Gly Gly Asp Leu Gly Glu Ala Ile Arg Glu Gln
        195                 200                 205

Pro Glu Gln Ala Arg Leu Ala Leu Thr Leu Ala Ala Ala Glu Ser Glu
    210                 215                 220

Arg Phe Val Arg Gln Gly Thr Gly Asn Asp Glu Ala Gly Ala Ala Asn
225                 230                 235                 240

Gly Pro Ala Asp Ser Gly Asp Ala Leu Leu Glu Arg Asn Val Pro Thr
                245                 250                 255

Gly Ala Glu Phe Leu Gly Asp Gly Gly Asp Val Ser Phe Ser Thr Arg
            260                 265                 270

Gly Thr Gln Asn Trp Thr Val Glu Arg Leu Leu Gln Ala His Arg Gln
        275                 280                 285

Leu Glu Glu Arg Val Tyr Val Phe Val Gly Tyr His Gly Thr Phe Leu
    290                 295                 300

Glu Ala Ala Gln Ser Ile Val Phe Gly Gly Val Arg Ala Arg Ser Gln
305                 310                 315                 320

Asp Leu Asp Ala Ile Trp Arg Gly Phe Tyr Ile Ala Gly Asp Pro Ala
                325                 330                 335

Leu Ala Tyr Gly Tyr Ala Gln Asp Gln Glu Pro Asp Ala Arg Gly Arg
            340                 345                 350

Ile Arg Asn Gly Ala Leu Leu Arg Val Tyr Val Pro Arg Ser Ser Leu
        355                 360                 365

Pro Gly Phe Tyr Arg Thr Ser Leu Thr Leu Ala Ala Pro Glu Ala Ala
    370                 375                 380

Gly Glu Val Glu Arg Leu Ile Gly His Pro Leu Pro Leu Arg Leu Asp
385                 390                 395                 400

Ala Ile Thr Gly Pro Glu Glu Glu Gly Gly Arg Leu Glu Thr Ile Leu
                405                 410                 415

Gly Trp Pro Leu Ala Glu Arg Thr Val Val Ile Pro Ser Ala Ile Pro
            420                 425                 430

Thr Asp Pro Arg Asn Val Gly Gly Asp Leu Asp Pro Ser Ser Ile Pro
        435                 440                 445

Asp Lys Glu Gln Ala Ile Ser Ala Leu Pro Asp Tyr Ala Ser Gln Pro
    450                 455                 460

Gly Lys Pro Pro Arg Glu Asp Leu Lys
465                 470

<210> 47
<211> 110
<212> PRT
<213> Artificial

<220>
<223>  synthetic

<400>  47

Asp Ile Gln Met Thr Gln Ser Ser Ser Ser Phe Ser Val Ser Leu Gly
1               5                   10                  15

Asp Arg Val Thr Ile Thr Cys Lys Ala Ser Glu Asp Ile Tyr Asn Arg
            20                  25                  30

Leu Ala Trp Tyr Gln Gln Lys Pro Gly Asn Ala Pro Arg Leu Leu Ile
        35                  40                  45

Ser Gly Ala Thr Ser Leu Glu Thr Gly Val Pro Leu Arg Phe Ser Gly
    50                  55                  60

Ser Gly Ser Gly Lys Asp Tyr Thr Leu Ser Ile Thr Leu Ser Leu Gln
65                  70                  75                  80

Thr Glu Asp Ile Ala Thr Tyr Tyr Cys Gln Gln Tyr Trp Ser Thr Pro
                85                  90                  95

Trp Thr Trp Thr Phe Gly Cys Gly Thr Lys Leu Glu Ile Lys
            100                 105                 110

<210>  48
<211>  27
<212>  DNA
<213>  Artificial

<220>
<223>  primer

<400>  48

agaaagacat gggtctggaa atggatg 27

<210> 49
<211> 26
<212> DNA
<213> Artificial

<220>
<223> primer

<400> 49
tctagaaaga catggcctgg aaacag 26

<210> 50
<211> 28
<212> DNA
<213> Artificial

<220>
<223> primer

<400> 50
gactgaactc agccaaggag ccagagtt 28

<210> 51
<211> 20
<212> DNA
<213> Artificial

<220>
<223> primer

<400> 51
cccaacatgg atcaggaact 20

<210> 52
<211> 21
<212> DNA

<213>  Artificial

<220>
<223>  primer

<400>  52
caccatggag ttacttttga g                                                21


<210>  53
<211>  35
<212>  DNA
<213>  Artificial

<220>
<223>  primer

<400>  53
cagaggcctg aacattgtct ggagtggatt ggaag                                 35


<210>  54
<211>  35
<212>  DNA
<213>  Artificial

<220>
<223>  primer

<400>  54
cttccaatcc actccagaca ctgttcaggc ctctg                                 35


<210>  55
<211>  38
<212>  DNA
<213>  Artificial

<220>
<223>  primer

<400>  55

gtccgccagg ctccaacgaa gtgtctggag tgggtcgc 38

<210> 56
<211> 38
<212> DNA
<213> Artificial

<220>
<223> primer

<400> 56
gcgacccact ccagacactt cgttggagcc tggcggac 38

<210> 57
<211> 41
<212> DNA
<213> Artificial

<220>
<223> primer

<400> 57
taagaaggag atatacatat ggaggttcag ctgcagcagt c 41

<210> 58
<211> 44
<212> DNA
<213> Artificial

<220>
<223> primer

<400> 58
gccctcggga cctccggaag cttttgagga gactgtgaga gtgg 44

<210> 59
<211> 33
<212> DNA

<213>  Artificial

<220>
<223>  primer

<400>  59
atacatatgg aggtgcagct ggtggagtct ggg                                    33


<210>  60
<211>  33
<212>  DNA
<213>  Artificial

<220>
<223>  primer

<400>  60
tccggaagct tttgaggaga cagtgactga agc                                    33


<210>  61
<211>  41
<212>  DNA
<213>  Artificial

<220>
<223>  primer

<400>  61
taagaaggag atatacatat ggacattgtg atgtcacaat c                           41


<210>  62
<211>  57
<212>  DNA
<213>  Artificial

<220>
<223>  primer

<400>  62

gctttgttag cagccgaatt cctatttgat ttccagcttg gtgccacaac cgaacgt 57

<210> 63
<211> 35
<212> DNA
<213> Artificial

<220>
<223> primer

<400> 63
atacatatgg acattgtgat gcccaatctc catcc 35